# EUROPEAN PATENT SPECIFICATION

(11) **EP 4 401 696 B1**
(45) Date of publication and mention of the grant of the patent: **27.08.2025**
(21) Application number: 22785895.8
(22) Date of filing: 13.09.2022
(51) Int. Cl.: A61H 9/00, A61H 23/02

(54) **SYSTEM AND APPARATUS TO APPLY VIBRATION, THERMAL AND COMPRESSIVE THERAPY**
SYSTEM UND VORRICHTUNG ZUR ANWENDUNG VON VIBRATIONS-, WÄRME- UND KOMPRESSIONSTHERAPIE
SYSTÈME ET APPAREIL POUR APPLIQUER UNE THÉRAPIE PAR VIBRATION, THERMIQUE ET PAR COMPRESSION

(30) Priority: 13.09.2021 US 202163243546 P
(43) Date of publication of application: 24.07.2024
(73) Proprietor: Hyperice IP SubCo, LLC, Irvine, CA 92618 (US)
(72) Inventor: MARTON, Robert, Irvine, California 92618 (US); KATZ, Anthony, Irvine, California 92618 (US); KERTH, Trevor Austin, Irvine, California 92618 (US); EDWARDS, Robert Glen, Irvine, California 92618 (US); NORTHRUP, John, Irvine, California 92618 (US); AGUIAR, Alexander Joseph, Irvine, California 92618 (US)
(74) Representative: D Young & Co LLP
(86) International application number: PCT/US2022/076374
(87) International publication number: WO 2023/039608

(56) References cited:
- DE-U1- 202019 001 024
- US-A1- 2019 015 295
- US-A1- 2021 275 390

## Description

### CROSS-REFERENCE TO RELATED APPLICATIONS

This application claims priority to and the benefit of U.S. Provisional Patent Application No. 63/243,546 titled "SYSTEM AND APPARATUS TO APPLY VIBRATION, THERMAL AND COMPRESSIVE THERAPY" and filed on September 13, 2021.

### TECHNICAL FIELD

The present invention is directed to the field of therapeutic devices, and, more particularly, is directed to the field of devices that provide vibration, thermal and compression to selected portions of a body.

### BACKGROUND

The applications of vibration and heat to tired and injured tissues are known to be therapeutic to the tissues. Various devices have been used to provide vibration, to provide heat or to provide a combination of vibration and heat. Many of the devices require continual manual application of the device. Other devices are configured to provide vibration, heat, or both vibration and heat to specific locations of the body by attachment to the location. Such devices require a person to purchase a different version of the device for each body location requiring therapy.

### SUMMARY

A need exists for a therapeutic vibration, thermal and compression apparatus that can be attached to different locations on a body without requiring a different device configuration for each location.

A device for applying vibration, thermal and compressive therapy is disclosed. In some embodiments, the device can include a top layer and a bottom layer. In some embodiments, the bottom layer of the device can be adapted to contact a body surface of a user. In some embodiments, the device can include a therapeutic element. In some embodiments, the therapeutic element can be disposed between the top layer and the bottom layer. In some embodiments, the therapeutic element can include a vibration component, a thermal component and a compression component. In some embodiments, upon activation of the therapeutic element, the vibration component applies a vibration force, the thermal component applies a thermal therapy, and the compression component applies a compressive force.

**In** some embodiments, the top layer can include a flexible, elastic material. In some embodiments, the bottom layer can include an inelastic material. In some embodiments, the inelastic material can include a molded silicone. In some embodiments, the compression component can include an inflatable bladder. In some embodiments, the device further includes an air compressor adapted to selectively inflate the inflatable bladder. In some embodiments, the air compressor can be disposed within a control module. In some embodiments, the compression component can be bonded to the bottom layer. In some embodiments, the compression component can be bonded to the bottom layer solely at the perimeter of the bottom layer. In some embodiments, one or more of the vibration component, the thermal component and the compression component are the same component. In some embodiments, upon activation of the therapeutic element, the compression component curves to more closely conform to the bottom layer.

One aspect of the embodiments disclosed herein is a system that applies compression, vibration and heat to a body part of a person. The system includes a portable vibration and heat generation apparatus having a flexible support platform and a bag-like enclosure extending from the support platform. A cylindrical control unit is mounted to the support platform and extends perpendicularly from the support platform. The control unit has a diameter of between 50 millimeters and 100 millimeters. The control unit houses electronic circuitry and at least one battery. Four vibration pods extend from the support platform into the bag-like structure. The bag-like structure also houses a heat generation unit. The control unit extends through a circular bore in a compression wrap. The compression wrap is securable to a body part with a distal wall of the bag-like enclosure against the body part. The system selectively applies vibration, heat or a combination of vibration and heat to the body part.

Another aspect of the embodiments disclosed herein is a portable vibration and heat generation apparatus. The apparatus comprises a flexible support platform, a cylindrical control unit, a plurality of vibration pods, a heat generation unit, and a bag-like enclosure. The cylindrical control unit is mounted to a central portion of the support platform and extends perpendicularly from the support platform in a first direction. The control unit has a diameter of between 50 millimeters and 100 millimeters. The control unit houses electronic circuitry and at least one battery. The plurality of vibration pods are attached to the flexible support platform. Each vibration pod extends from the support platform in a second direction, which second direction is opposite the first direction, the vibration pods are electrically connected to the control unit. The heat generation unit is positioned below the vibration pods. The heat generation unit electrically connected to the control unit. The bag-like enclosure is attached to the support platform and encloses the plurality of vibration pods and the heat generation unit. The bag-like enclosure has a distal wall. The heat generation unit is positioned adjacent to the distal wall. In certain embodiments, each vibration pod includes an electrical motor having a shaft coupled to an eccentric mass. In certain embodiments, four vibration pods are arranged generally symmetrically about thecylindrical control unit. In certain embodiments, the heat generation unit comprises at least one resistance heating wire secured to a flexible sheet. The resistance heating wire generates heat when a current flows through the heating wire. In certain embodiments, the heat generation unit is operable at at least a first temperature setting, a second temperature setting and a third temperature setting. In certain embodiments, the control unit is responsive to a signal received via a wireless communication interface. For example, in certain embodiments, the wireless communication interface is a Bluetooth interface. In certain embodiments, the flexible support platform, the control unit and the bag-like enclosure have sizes and shapes selected to cause the vibration and heat generation apparatus to resemble a therapeutic ice bag.

Another aspect of the embodiments disclosed herein is a system for applying compression, vibration and heat to a body part of a person. The system comprises a portable vibration and heat generation apparatus and a compression wrap. The portable vibration and heat generation apparatus comprises a flexible support platform, a cylindrical control unit, a plurality of vibration pods, a heat generation unit and a bag-like enclosure. The cylindrical control unit is mounted to a central portion of the support platform and extends perpendicularly from the support platform in a first direction. The control unit has a diameter of between 50 millimeters and 100 millimeters. The control unit houses electronic circuitry andat least one battery. The plurality of vibration pods are attached to the flexible support platform. Each vibration pod extends from the support platform in a second direction, which second direction is opposite the first direction. The vibration pods are electrically connected to the control unit. The heat generation unit is positioned distal to the vibration pods. The heat generation unit is electrically connected to the control unit. The bag-like enclosure is attached to and extends distally from the support platform. The bag-like enclosure encloses the plurality of vibration pods and the heat generation unit. The bag-like enclosure has a distal wall. The heat generation unit is positioned adjacent to the lower wall. The compression wrap comprises a unitary sheet of elastic material having a central body with straps extending therefrom. The central body includes at least one bore that receives the cylindrical control unit of the portable vibration and generation apparatus therethrough. The straps of the compression wrap are positionable with respect to the body part of the person to secure the distal wall of the bag-like enclosure of the portable vibration and generation apparatus against the body part to apply heat from the heat generation unit to the body part and to apply vibrationfrom the vibration pods to the body part. In certain embodiments, the flexible support platform, the control unit and the bag-like enclosure have sizes and shapes selected to cause the portable vibration and heat generation apparatus to resemble a therapeutic ice bag.

Another aspect of the embodiments disclosed herein is a system for applying a combination of compression, vibration and heat to a body part of a person. The system comprises a portable vibration and heat generation apparatus and a compression wrap. The portable vibration and heat generation apparatus includes a flexible support platform, a bag-like enclosure, a cylindrical control unit, a plurality of vibration pods and a heat generation unit. The flexible support platform has an outer perimeter. The bag-like enclosure has a perimeter attachedto the outer perimeter of the support platform. The bag-like enclosure extends distally from the support platform in a first direction to a distal wall. The cylindricalcontrol unit is mounted to the support platform and extends perpendicularly proximally from the support platform in a second direction opposite the first direction. The control unit has a diameter of between 50 millimeters and 100 millimeters. The control unit houses electronic circuitry and at least one battery. The control unit includes a panel having a plurality of touch responsive areas thereon to receive commands to control the electronic circuitry. Each vibration pod has at least a portion extending from the support platform in the first direction andenclosed within the bag-like structure. The heat generation unit is enclosed withinthe bag-like structure and is positioned proximate to the distal wall of the bag-likestructure. The compression wrap has a bore formed therethrough. The cylindrical control unit of the portable vibration and heat generation apparatus extends through the bore. The compression wrap is securable to a body part with the distal wall of the bag-like enclosure against the body part. In certain embodiments, the flexible support platform, the control unit and the bag-like enclosure have sizes and shapes selected to cause the portable vibration and heat generation apparatus to resemble a therapeutic ice bag.

### BRIEF DESCRIPTION OF THE DRAWINGS

The foregoing aspects and other aspects of the disclosure are described indetail below in connection with the accompanying drawings in which:
FIG. 1 illustrates a top perspective view of a vibration and heat generation apparatus that can be applied to different locations of body, the apparatus including a vibration generation mechanism and a heat generation mechanism housed within a flexible enclosure, the apparatus further including a control unit housed within a generally cylindrical enclosure and extending from an upper flexible support structure;
Fig. 2 illustrates a bottom perspective view of the vibration and heat generation apparatus of FIG. 1, the view showing the flexible bag-like lower housing extending from the upper flexible support structure of FIG. 1;
FIG. 3 illustrates a top plan view of the vibration and heat generation apparatus of FIG. 1;
FIG. 4 illustrates a bottom plan view of the vibration and heat generation apparatus of FIG. 1;
FIG. 5 illustrates a front elevational view of the vibration and heat generation apparatus of FIG. 1;
FIG. 6 illustrates a right side elevational view of the vibration and heat generation apparatus of FIG. 1;
FIG. 7 illustrates an exploded perspective view of the vibration and heat generation apparatus of FIG. 1;
FIG. 8 illustrates a front elevational cross-sectional view of the vibration and heat generation apparatus of FIG. 1 taken along the line 8--8 in FIG. 3;
FIG. 9 illustrates an upper perspective view of one of the four vibrational pods of the vibration and heat generation apparatus of FIG. 1;
FIG. 10 illustrates an exploded perspective view of the vibrational pod of FIG. 9 showing the upper surfaces of the components;
FIG. 11 illustrates an exploded perspective view of the vibrational pod of FIG. 9 with the view of FIG. 10 inverted to show the lower surfaces of the components;
FIG. 12 illustrates an upper perspective view of the lower cover of the vibrational pod of FIG. 9 rotated by a small angle to show addition features of the cavity of the lower cover;
FIG. 13 illustrates an exploded upper perspective view of the heating pad of the vibration and heat generation apparatus showing the heating elements, thetemperature sensor and the thermal cutoff switch on the lower sheet of the heating pad;
FIG. 14 illustrates a plan view of the electrical heating wire on the lower sheet of the heating pad of FIG. 13;
FIG. 15 illustrates an exploded upper perspective view of the cylindrical control unit of the vibration and heat generation apparatus;
FIG. 16 illustrates an exploded perspective view of the cylindrical control unit of FIG. 15 with the view of FIG. 15 inverted to show the lower surfaces of the components of the control unit;
FIG. 17 illustrates a top plan view of the touch panel control interface positioned on the upper end of the cylindrical control unit of FIG. 16;
FIG. 18 illustrates a block diagram of the electrical and electronic circuitry of the vibration and heat generation apparatus of FIG. 1;
FIG. 19 illustrates an elevational view of the vibration and heat generation apparatus of FIG. 1 showing the flexing of the upper support structure and the bag- like lower housing to conform the apparatus to a cylindrical object such as a human limb;
FIG. 20 illustrates an elevational view of a compression wrap configured tobe attached to a person proximate to the person's hip, the compression wrap including a circular bore to receive the control unit of the vibration and heat generation unit of FIG. 1;
FIG. 21 illustrates an elevational view of a compression wrap configured to be attached to a person proximate to the person's knee, the compression wrap including a circular bore to receive the control unit of the vibration and heat generation unit of FIG. 1;
FIG. 22 illustrates an elevational view of a compression wrap configured tobe attached to a person proximate to the person's left shoulder, the compression wrap including a circular bore to receive the control unit of the vibration and heat generation unit of FIG. 1;
FIG. 23 illustrates an elevational view of a compression wrap configured tobe attached to a person proximate to the person's right shoulder, the compression wrap including a circular bore to receive the control unit of the vibration and heat generation unit of FIG. 1;
FIG. 24 illustrates an elevational view of a compression wrap configured tobe attached to a person proximate to the person's left shoulder, the compression wrap including a first circular bore and a second circular bore, each circular bore configured to receive the control unit of a respective vibration and heat generation unit of FIG. 1;
FIG. 25 illustrates an elevational view of the compression wrap of FIG. 21 with the control unit of the vibration and heat generation unit of FIG. 1 extending through the circular bore;
FIG. 26 illustrates a perspective view of the compression wrap and vibration and heat generation unit of FIG. 25 secured to a person's knee;
FIG. 27 illustrates an elevation view of the compression wrap of FIG. 24 with a control unit of a first vibration and heat generation unit of FIG. 1 extending through the first circular bore and with a control unit of a second vibration and heat generation unit of FIG. 1 extending through the second circular bore;
FIG. 28 illustrates a front perspective view of the compression wrap and the two vibration and heat generation units of FIG. 27 secured to the left shoulder of aperson, the view showing the first vibration and heat generation unit positioned proximate to the front of the person's left shoulder;
FIG. 29 illustrates a front perspective view of the compression wrap and the two vibration and heat generation units of FIG. 27 secured to the left shoulder of aperson, the view showing the second vibration and heat generation unit positioned proximate to the rear of the person's left shoulder;
FIG. 30 illustrates an example exploded view of a device for applying vibration, thermal and compressive therapy, according to various embodiments;
FIG. 31 illustrates an example control module, according to various embodiments;
FIG. 32 illustrates an example exploded view of the control module of FIG. 31; and
FIG. 33 illustrates a block diagram of an example computer system, according to various embodiments.

### DETAILED DESCRIPTION

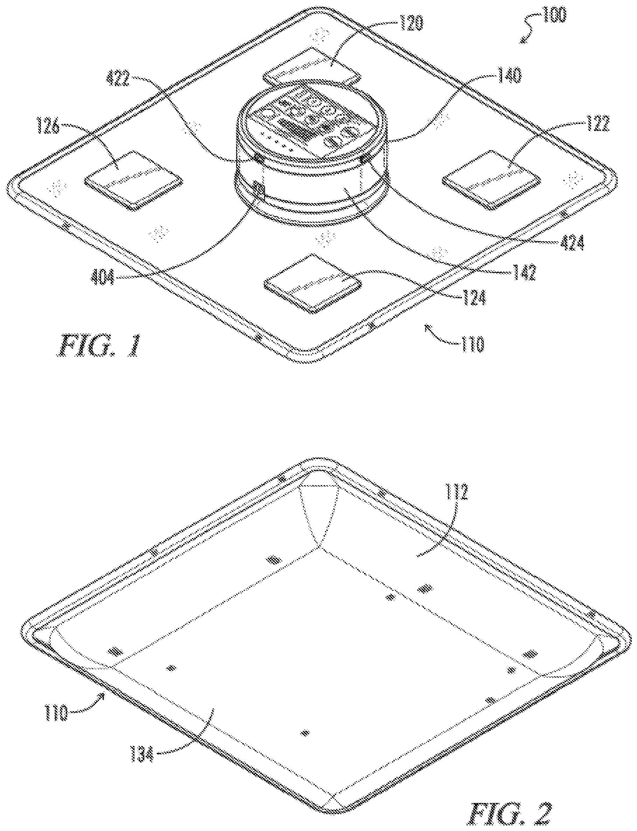

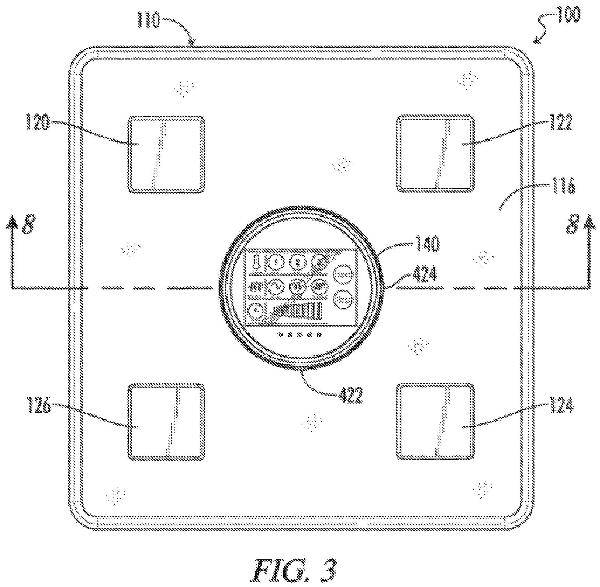

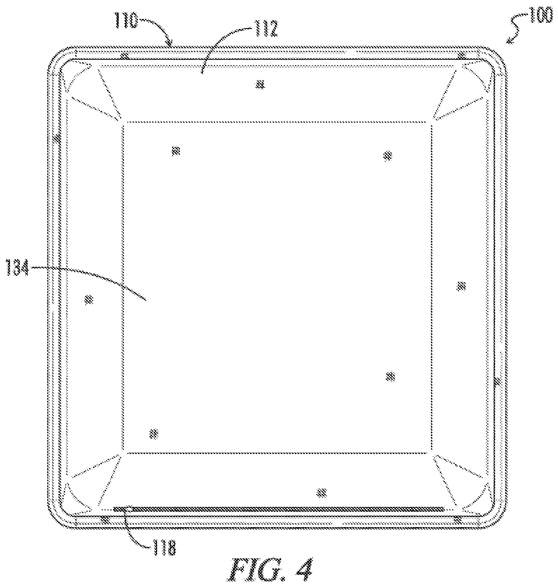

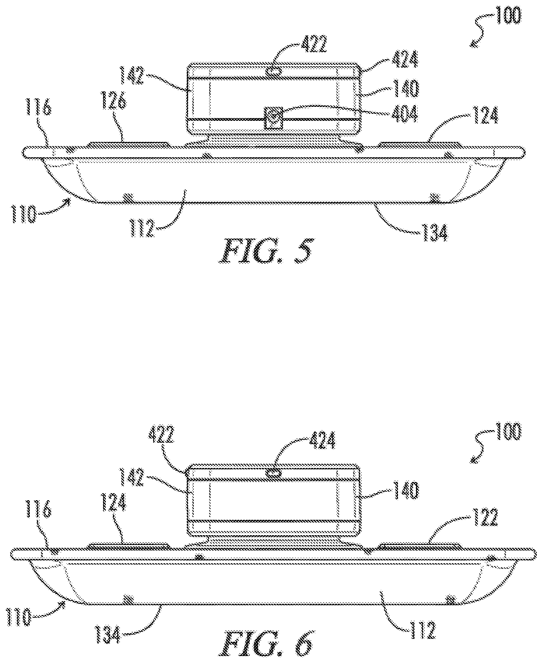

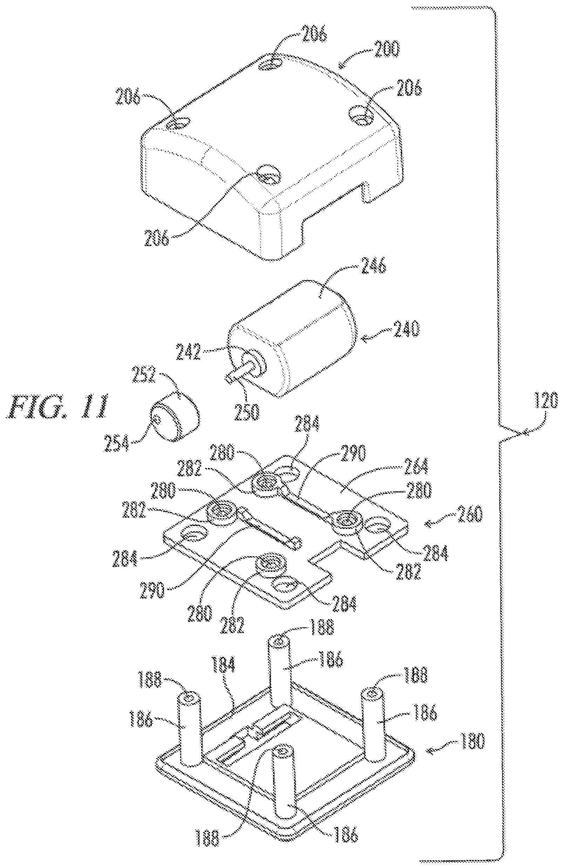

A vibration and heat generation apparatus 100 is illustrated in FIGS. 1-18. As described below, the vibration and heat generation apparatus can be applied to different locations of body. The apparatus can apply vibration to a selected location of the body, can apply heat to the selected location of the body, and can apply a combination of vibration and heat to the selected location of the body. The apparatus is particularly adapted to be used with compression wraps, which are also described below.

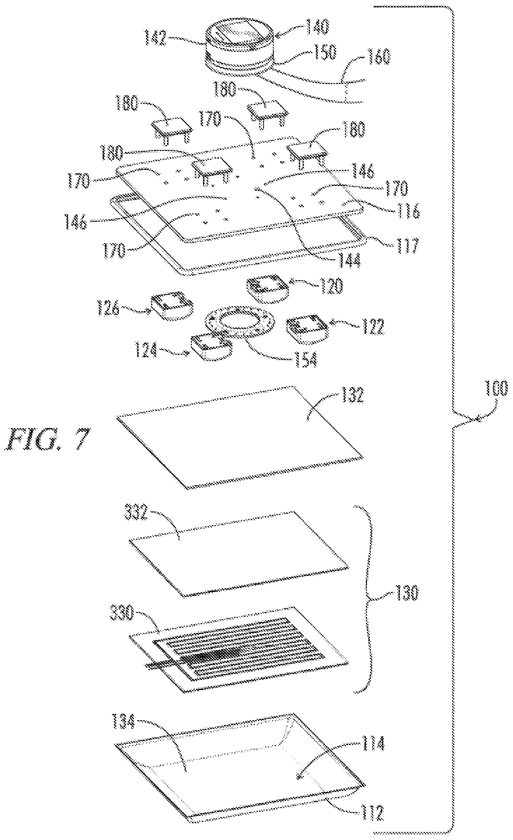

The vibration and heat generation apparatus 100 includes an enclosure 110. The enclosure comprises a lower bag-like structure 112 that houses an inner cavity 114 (FIG. 7). The lower bag-like structure is secured to anupper support structure 116 and extends distally from the upper support structure. In the illustrated embodiment, the lower bag-like structure comprises a strong elastomeric fabric such as, for example, a polyester-polyurethane copolymer fiber commonly referred to as spandex. In the illustrated embodiment, the upper support structure comprises a strong, flexible material. For example, the material may be an elastomeric material such as neoprene. Other strong, flexible materials can also be used. In the illustrated embodiment, the upper support structure has a width of approximately 10.25 inches (approximately 26.1 centimeters), a length of approximately 10.75 inches (approximately 27.3 centimeters) and a thickness of approximately 5 millimeters.

In the illustrated embodiment, the lower structure 112 is sewn to the upper support structure 116 along the four sides of the upper support structure. The seam between the two structures may be reinforced with bias tape 117 or other material as shown. In the illustrated embodiment, a zipper 118 is sewn into the lower structure to allow selective access to the cavity in the lower structure for initial installation of the components described below. The zipper is positioned near one edge of the lower structure as shown. The zipper is attached in such a manner that the edges of the fabric of the lower structure proximate to the two sides of the zipper are almost touching to substantially hide the underlying zipper from view. The material comprising the lower structure has generally rectangular dimensions sufficiently larger than the corresponding dimensions of the upper support structure such that the lower structure forms the inner cavity 114 with a sufficient depth relative to the upper support structure to accommodate a plurality of vibration elements (e.g., a first vibration pod 120, a second vibration pod 122, a third vibration pod 124 and a fourth vibration pod 126). The inner cavity further accommodates at least one heat generation unit 130. The heat generator is mechanically and thermally buffered from the vibration pods by a layer 132 of flexible foam.

As used herein, "bag-like structure" refers to various shapes the lower structure 112 may have when in use because the lower structure comprises a fabric material that is readily deformable to conform the material to irregular shapes. When the lower structure and the upper support structure 116 are resting on a flat surface, the lower structure has a selected general shape defined by its outer dimensions such that a flexible distal (e.g., lowermost in the illustrated orientation) wall 134 of the lower structure is generally parallel to the upper support structure. The actual shape of the lower structure varies in response to the current shape of the upper support structure. For example, when the outer edges of the upper support structure are bent downward, the distal wall of the lower structure may sag away from the upper support structure. On the other hand, when the upper support structure is positioned on a person's knee or other curved body part, the flexible distal wall of the lower structure easily deforms to conform to the irregular curvature of the body part.

A control unit 140 extends proximally (e.g., upward in the illustrated orientation) from a proximal (top) surface of the upper support structure 116. The control unit is housed within a generally cylindrical enclosure 142. As shown in the exploded view (FIG. 7), the upper support structure includes a though bore 144 that is positioned close to the center of the upper support structure. The through bore has a sufficient size to accommodate a plurality of power wires (e.g., twelve wires), which are discussed below. For example, the through bore may have a diameter between 0.25-0.6 cm ( 0.1 inch and 0.25 inch). The control unit together with the enclosure 110, comprising the upper support structure and the lower structure 112, results in the vibration and heat generation apparatus 100 having an overall size and shape resembling a conventional flattened ice bag.

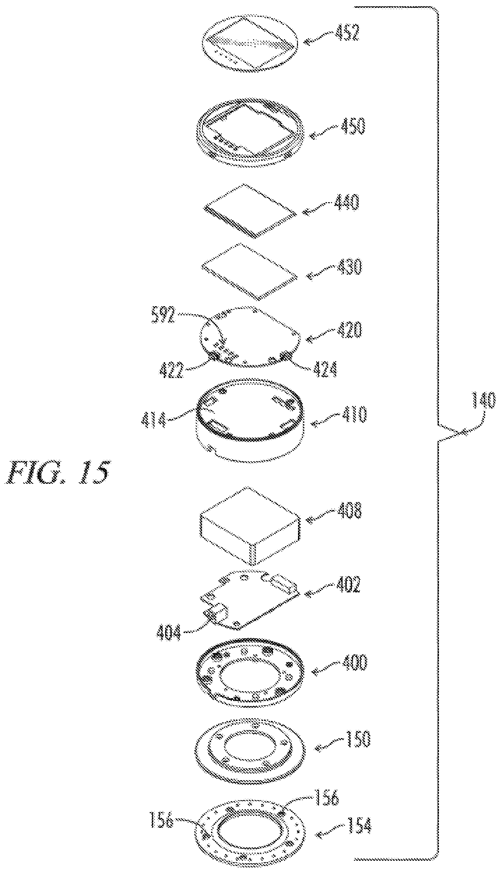

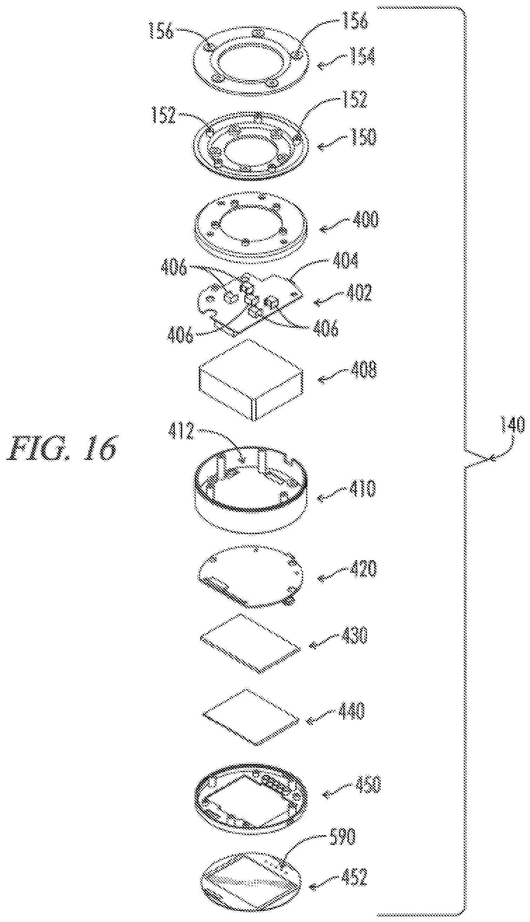

[As shown in FIG. 7, the through bore 144 in the upper support structure 116 is surrounded by a plurality of mounting holes 146 formed through the upper support structure. For example, five mounting holes are equally spaced about a circle centered at the center of the upper support structure. In one embodiment, the circle has a diameter of approximately 3.05 inches. The cylindrical enclosure has an annular lower flange 150 that is positioned concentrically with respect to the cylindrical bore. The lower flange includes a plurality of threaded bores (e.g., five bores) 152 (FIG. 16) that are aligned with the mounting holes in the upper support structure. An annular compression flange 154 is mounted below the upper support structure. The compression flange includes a corresponding plurality of unthreaded bores (e.g., five bores) 156 (FIGS. 15 and 16) aligned with the mounting holes and aligned with the threaded bores of the annular lower flange. A corresponding plurality of screws (not shown) pass through the unthreaded bores of the compression flange and engage the threaded bores of the lower flange. As the screws are tightened, an annular portion of the upper mounting surface surrounding the central cylindrical bore is squeezed between the compression flange and the lower flange to secure the cylindrical enclosure to theupper support structure. It should be understood that the screws may be machine screws that engage pre-threaded bores in the lower flange or may be self- threading screws that create threads in the bores of the lower flange when the compression flange and the lower flange are first interconnected.

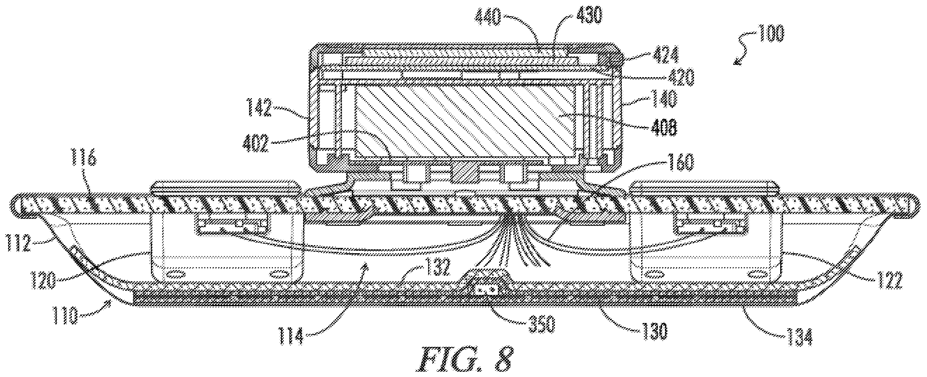

As further shown in FIG. 8, a plurality of electrical wires 160 extend from the lower portion of the cylindrical enclosure 142 of the control unit 140 and through the through bore 144 (FIG. 7) of the upper support structure 116. Additional structural and operational features of the control unit are described below.

The upper support structure 116 further includes a plurality of pod mounting bores 170 that extend through the upper support structure. In the illustrated embodiment, the upper support structure includes four sets of pod mounting bores. Each set of mounting bores comprises four bores arranged in a generally square pattern with a respective bore at the vertex of the square pattern. For example, in one embodiment, the bores in each set of positioned approximately 30 millimeters (approximately 1.2 inches) apart and have diameters of approximately 5 millimeters (approximately 0.2 inch). In the illustrated embodiment, each set of pod mounting bores is centered at selected distances from the center of the upper support structure. For example, the center of a rear left set is positioned approximately 7.2 cm (2.85 inches) to the left of the center of the upper support structure and approximately 7.2 cm (2.85 inches) toward the rear relative to the center of the upper support structure. In the illustrated embodiment, the sets of pod mounting bores are positioned substantially symmetrically with respect to the center of the upper support structure such that the center of each set is approximately the same distance from the center of the upper support structure. In other embodiments, the sets of mounting bores may be positioned differently from front to rear than from left to right, particularly if the upper support structure has a non-square upper surface. Note that as used herein, left and right, front and rear, and top and bottom are used to indicate positions relative to the drawings with the exposed upper surface of the upper support structure designated as the "top" or "proximal" surface. The apparatus may be used in many different orientations wherein the upper surface of the upper support structure may be oriented outward, downward or the like.

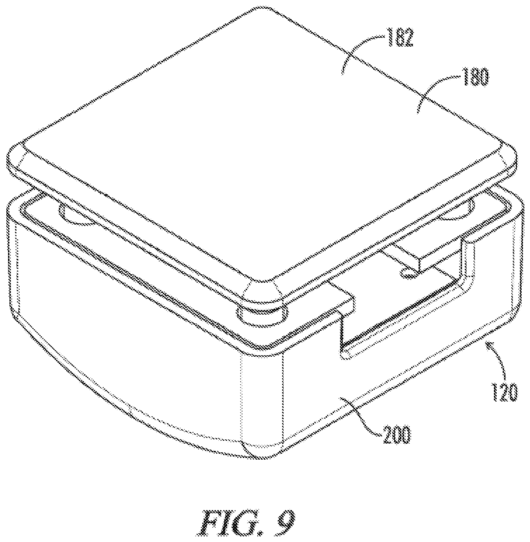

The first vibration pod 120 is shown in more detail in FIGS. 9-11. The other three vibration pods 122, 124, 126 are identical or are substantially identical. The first vibration pod includes an upper cover 180. In the illustrated embodiment, a top surface 182 of the upper cover is square or substantially square with each side of the square having a length of approximately 45 millimeters). The upper cover has a thickness of approximately 4.25 millimeters to a lower surface 184. Four protrusions 186 extend from the lower surface of the upper cover. Each protrusion has a diameter selected such that each protrusion fits through a selected one of the mounting bores 170 in the rear left set of mounting bores. For example, in the illustrated embodiment, the protrusions have a diameter of approximately 5 millimeters. Each protrusion has a length of approximately 16.5 millimeters. The end of each protrusion opposite the top of the upper cover has a central bore 188 that may be threaded to receive a machine screw (not shown). Alternatively, the central bore may be threadable to receive a self-taping screw.

The first vibration pod 120 includes a lower cover 200 having a central cavity 202. The lower cover has a generally square upper surface 204 surrounding the central cavity. In the illustrated embodiment, the peripheral dimensions of theupper surface of the lower cover generally correspond to the peripheral dimensions of the upper cover 180. The lower cover has an arcuate lower surface having four through bores 206 formed therein. The through bores are spaced apart by distances corresponding to the spacing of the protrusions 186 of the upper cover 180. The through bores are counterbored with respect to the lower cover to receive the heads of the screws (not shown) that secure the lower cover to the upper cover.

A lower inner surface 210 of the lower cover 200 corresponds to the lower surface of the central cavity 202. Each of the through bores 206 is surrounded bya respective inner protrusion 212 that extends from the lower inner surface of the central cavity. The top surface of each inner protrusion has a respective counterbore 214 that surrounds the through bore and extends a selected distance into the protrusion. The diameter of each counterbore is selected to correspondto the outer diameter of the protrusions 186 extending from the top cover 180 (e.g., approximately 5 millimeters in the illustrated embodiment) so that each protrusion of the top cover fits snugly into the respective counterbore of one of the inner protrusions of the lower cover. The depth of the counterbore in each inner protrusion in the central cavity is selected such that when the protrusions of the top cover are engaged with the counterbores, the lower surface 184 of the top cover is spaced apart from the upper surface 204 of the bottom cover by a distance less than the thickness of the upper support structure 116. For example, in the illustrated embodiment, the two surfaces are spaced apart by approximately 1.85 millimeters, which is substantially less than the thickness (e.g., approximately 5 millimeters) of the upper support structure. Thus, when the top cover is securedto the bottom cover by the four screws (not shown) passing through the through bores 206 of the lower cover and engaging the central bores 188 of protrusions extending from the upper cover, the portions of the upper support structure in contact with the upper cover and the lower cover are squeezed between the two covers to secure the first vibration pod 120 to the upper supportstructure. The other three vibration pods 122, 124, 126 are secured to the uppersupport structure in a like manner.

The lower inner surface 210 of the lower cover 200 includes a first motor bearing support 230 and a second motor bearing support 232. Each motor bearing support is sized and positioned to receive a respective motor bearing as described below. The lower inner surface further includes three raised ribs 234 positioned between the first and second bearing supports. Each rib has a respective upper surface positioned a selected distance from the lower inner surface.

The first bearing support 230 includes a generally semicircular upper surface sized to receive a front bearing 242 of a motor 240. The second bearing support 232 includes a generally semicircular upper surface sized to receive a rear bearing 244 of the motor. The motor has a generally horizontal lower surface 246that rests on the three raised ribs 234 when the bearings of the motor are positioned in the respective bearing supports. The motor also has a generally horizontal upper surface 248, which is parallel to the upper surface in the illustrated embodiment. The motor includes a shaft 250. A front portion of the shaft extendsfrom the front bearing to support an eccentric mass 252. The eccentric mass is positioned within an unobstructed portion of the inner cavity and is able to move freely within the portion of the cavity when the shaft of the motor is rotated.

The lower cover 200 further includes a motor clamp plate 260 having an upper surface 262 and a lower surface 264. The motor clamp plate rests upon four clamp plate support protrusions 270 that extend upward from the lower innersurface 210. Each clamp plate support protrusion has a respective central bore 272. Each central bore may be threaded to receive the threads of a machinescrew (not shown). Alternatively, each central bore may be threadable by a self-tapping screw.

The motor clamp plate 260 is sized to fit within the lower cover 200 and to rest upon the clamp plate support protrusions 270. The motor clamp plate includes four plate mounting through bores 280 that are aligned with the central bores of the clamp plate support protrusions. Each plate mounting through bore is counterbored on the upper surface 262 of the motor clamp plate so that the headsof the machine (or self-tapping) screws (not shown) do not extend above the upper surface of the motor clamp plate.

The lower surface 264 of the motor clamp plate 260 includes a respective protrusion 282 surrounding each plate mounting through bore 280. Each protrusion extends a short distance (e.g., approximately 2 millimeters; approximately 0.08 inch) below the lower surface. Each protrusion is counterbored to have an inside diameter corresponding to the outside diameter of a clamp platesupport protrusion 270 (e.g., approximately 2.3 millimeters; approximately 0.09 inch in the illustrated embodiment). Thus, when the motor clamp plate is secured to the clamp plate protrusions, the motor clamp plate cannot shift laterally with respect to the lower cover.

The motor clamp plate 260 further includes four clearance through bores 284, which are positioned and sized to provide clearance for the four protrusions 186 that extend from the lower surface 184 of the upper cover 180. For example, in the illustrated embodiment, the clearance through bores have diameters of slightly greater than approximately 5 millimeters (approximately 0.2 inch) to provide a snug fit with respect to the protrusions.

The motor clamp plate 260 includes two motor engagement ribs 290 that extend from the lower surface 264. The engagement ribs are positioned to engage the generally horizontal upper surface 248 of the motor 240 when the motor clamp plate is positioned on the lower cover 200 of the first vibration pod 120. The thickness of each rib with respect to the lower surface of the motor clamp plate isselected such that when the motor clamp plate is fully secured by the four screws(not shown), the ribs are pressed against the horizontal upper surface of the motor. Accordingly, the motor is tightly secured between the ribs of the motor clamp plateand the three raised ribs 234 of the lower inner surface 210 of the lower cover 200.

In the illustrated embodiment, the motor 240 comprises a permanent magnet DC motor operating at approximately 5,300 revolutions per minute (RPM)from a 12-volt DC supply. In one embodiment, the motor comprises an FC130 style motor, which is commercially available from a number of sources. The motor draws approximately 0.09 Amperes at the rated RPM.

The motor 240 and the eccentric mass 252 together have an overall lengthof approximately 38 millimeters. The motor has an overall diameter of approximately 20.2 millimeters and is flattened to space the lower surface 246 and the upper surface 248 apart by approximately 15.4 millimeters.

The eccentric mass 252 is substantially cylindrical. The eccentric mass has an overall diameter of approximately 10 millimeters, and has a length along the shaft of the motor of approximately 7 millimeters. In the illustrated embodiment, the mass comprises powdered metal (e.g., iron), which is compacted to have a mass (weight) of approximately 3.5 grams. The eccentric mass is mounted on the shaft 250 of the motor 240 via a shaft bore 254 having a diameter of approximately

2.1 millimeters. In the illustrated embodiment, the shaft bore is offset from the center of the eccentric mass by approximately 2.2 millimeters to cause the mass to impart a vibration. The vibration is communicated from the shaft of the motor and through the bearings 242, 244 to bearing supports 230, 232 to cause the lower cover 200 of the vibration pod 120 to vibrate.

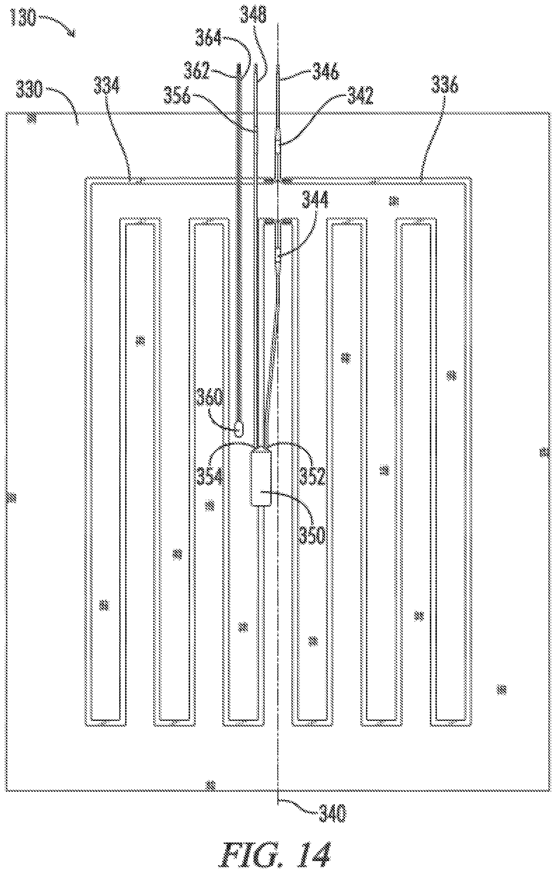

Each of the four vibration pods 120, 122, 124, 126 are electrically connected to the control unit as described below. As illustrated in FIG. 14, in the illustrated embodiment, the heat generation unit 130 comprises a first (lower) rectangular sheet of cloth 330 and a second (upper) rectangular sheet of cloth 332. Each sheet has outer dimensions of approximately 250 millimeters by approximately 200 millimeters. In the illustrated embodiment, each sheet comprises a 200g needle punch material (i.e., non-woven material formed by a conventional needle punching process) having a thickness of approximately 1.5 millimeters. The material has a density of approximately 200 grams per square meter. At least one electrical resistance wire is positioned between the two sheets. In the illustrated embodiment, a first resistance wire 334and a second resistance wire 336 are secured to the upper surface of the lower sheet by lock stitching (not shown) in a conventional manner. The resistance wires can also be secured to the upper sheet in a similar manner. In one embodiment,each resistance wire comprises a thin, flat resistance wire, such as, for example, a commercially available titanium resistance wire. In the illustrated embodiment, the cross-sectional dimensions of the resistance wires are selected to provide a resistance of approximately 16 ohms per meter. Each resistance wire has a length of approximately 1.25 meters such that each wire has a total resistance of approximately 20 ohms.

The two resistance wires 334, 336 form two maze-like patterns, which are substantially symmetric about a centerline 340 of the lower sheet 330. Each resistance wire extends from a first common terminal 342 to a second common terminal 344 such that the two segments are connected in parallel. The first common terminal of the resistance wires is connected directly to a first supply wire 346. The second common terminal of the resistance wires is connected to a second supply wire 348 via a thermal cutoff switch 350. The thermal cutoff switchhas a first terminal 352 connected to the second common terminal of the resistance wires and has a second terminal 354 connected to the second supply wire via a connector 356. The thermal cutoff switch 350 is normally closed such that the control unit 140 is electrically connected to the second common terminal 344 of the resistance wires 334, 336. The first common terminal 342 of the resistance wiresis always connected to the control unit. Thus, current is conducted from the first terminal around each of the first resistance wire and the second resistance wire inparallel. Since each resistance wire has a resistance of approximately 20 ohms, each resistance wire generates approximately 14 watts of heat at a voltage of approximately 16.8 volts. The two resistance wires generate a total of approximately 28 watts of heat.

The thermal cutoff switch 350 is set to open the circuit when the temperature proximate to the thermal cutoff switch exceeds approximately 80 degrees Celsius +/-5 degrees and to stay open until the temperature reduces to approximately 55degrees Celsius +/-10 degrees. In one embodiment, the thermal cutoff switch comprises a KLS-KSD9700 thermal fuse commercially available from Ningbo KLS Imp & Exp Co. Ltd. In Beilun Ningbo Zhejiang China. The thermal cutoff switch ispositioned across portions of the heating wire such that the thermal cutoff switch directly senses the temperature of the heating wire and disconnects the electricalpath well before the heat from the heating wire is communicated though the lowersheet and the material of the lower structure 112 to a user (not shown).

As further shown in FIGS. 14 and 15, a thermistor 360 is secured to the first (lower) sheet of cloth 330. The thermistor is also positioned near the center of thefirst sheet; however, the thermistor is positioned between two adjacent segmentsof the first resistance wire 334 rather than directly on the resistance wire. A first wire 362 and a second wire 364 extend from the thermistor and are connected tothe control unit 140. In one embodiment, the thermistor is a negative temperaturecoefficient (NTC) thermistor. For example, the thermistor may be an MF52-104F-3950-600L thermistor commercially available from Dongguan Xinxiang ElectronicTechnology Co., Ltd., in China. The thermistor has a resistance that varies over a wide temperature range. For example, at 55 degrees Celsius, the thermistor has a resistance of approximately 29,733 ohms; at 60 degrees Celsius, the thermistor has a resistance of approximately 24,753 ohms; and at 71 degrees Celsius, the thermistor has a resistance of approximately 16,794 ohms. The resistance of the thermistor is readily detectable in a conventional manner to determine when the temperature of the thermistor exceeds a selected temperature.

After the thermal cutoff switch 350 and the thermistor 360 are positioned on the first (lower) sheet 330, and after the first common terminal 342 is connected tothe first supply wire 346 and the second common terminal 344 is connected to a second supply wire 348, the second (upper) sheet 332 is secured to the first sheet. In the illustrated embodiment, the lower surface of the second sheet includes an adhesive to removably attach the second sheet to the first sheet.

As further shown in FIGS. 7 and 8, the layer 132 of flexible foam is positioned above the second (upper) sheet 332 between the second sheet and the vibration pods 120, 122, 124, 126 to partially buffer the vibrations provided by thevibration pods when operated as described below.

The structure of the control unit 140 is shown in more detail in FIGS. 15 and 16. As described above, the control unit includes the lower flange 150 and the removably attachable annular compression flange 154. The lower flange is connected to a lower body portion 400 of the control unit. The lower body portionsupports a first printed circuit board (PCB) 402.

The first PCB 402 includes an electrically and mechanically attached conventional charging jack 404, which extends through a notch in the wall of the lower body portion. The first PCB also includes a plurality of metal oxide semiconductor field effect transistors (MOSFETs) (not shown) that provide power to the vibration pods 120, 122, 124, 126 and to the heat generation unit 130 via a plurality of connectors 406. A lithium polymer (LiPo) battery 408 rests upon the first PCB and is electrically connected to the first PCB to receive charging energyvia the first PCB and to provide operational energy to the other components of the control unit. The lower body portion includes a central opening to allow wiring from the connectors to the vibration pods 120, 122, 124, 126 and to the heat generation unit 130 to pass therethrough.

A cylindrical middle body portion 410 is positioned over the first PCB 402 and the LiPo battery 408 and is secured to the lower body portion. A lower end 412 of the middle body portion is open. An upper end 414 of the middle body portion is generally closed; however, the upper end includes a plurality of through passages to allow wiring to pass through the upper end from the first PCB to a second PCB 420. The middle body portion also includes a notch to accommodate the charging jack 404.

The second PCB 420 rests on the upper end 414 of the middle body portion 410 and is secured to the upper end by suitable fasteners (not shown). The second PCB is electrically connected to the first PCB 402 via a plurality of wires (not shown). The second PCB receives power from the battery 406 via the first PCB 402. The second PCB also receives input power from the power input jack 404. The second PCB generates a battery charging voltage of approximately 16.8 volts, which is provided to the battery via the first PCB. The second PCB also generates a motor voltage of approximately 12 volts, which is provided to the first PCB as a motor driving voltage. The second PCB generates control signals to control the power applied to the vibration pods 120, 122, 124, 126 and to the heat generation unit 130. The control signals are applied to the MOSFETs (not shown) on the first PCB.

The second PCB 420 communicates with a liquid crystal display (LCD) panel and a touch panel (described below). The second PCB is electrically connected to a first pushbutton switch 422 and to a second pushbutton switch 424. The two switches are mounted on the printed circuit board in the illustrated embodiment. The first pushbutton switch is manually operable to turn the vibration and heat generation apparatus 100 on and off. The second pushbutton switch is manually operable to select between two brightness levels for the LCD display. Each brightness level corresponds to a respective operational mode for the touchpanel. The electronic circuitry on the second PCB and the two operational modesare described in more detail below.

An LCD panel 430 is positioned proximate to and electrically connected to the second PCB 420. For example, the LCD panel may be a "daughter board" mechanically connected to the second PCB via a connector (not shown). The LCD panel may also be connected to the second PCB via a plurality of electrical wires(not shown). The LCD panel is responsive to signals from the second PCB to generate signals to cause images to be displayed as described below.

A generally transparent touch panel 440 is positioned over the LCD panel 430. The touch panel generates signals resulting from manual manipulation of selected portions of the touch panel. The signals are provided to the second PCB. In certain embodiments, the LCD panel and the touch panel are provided incombination as a single integrated package. Such combinations are commercially available and are well understood. In the illustrated embodiment, the LCD panel and the display panel comprise a Model No. YH26167VNT display commercially available from Dongguan Quinniahong Electronic Technology Co., Ltd., in China.

An upper body portion 450 is positioned over the LCD panel 430, the touchpanel 440 and the second PCB 420. A middle section of the upper body portion is removed to expose the LCD touch panel such that the images displayed on theLCD touch panel are visible to a user and such that a user can access the surfaceof the LCD touch panel with the user's fingertips or with a suitable stylus. In the illustrated embodiment, a bezel 452 is positioned over the upper body portion to frame the active portions of the LCD panel and the touch panel.

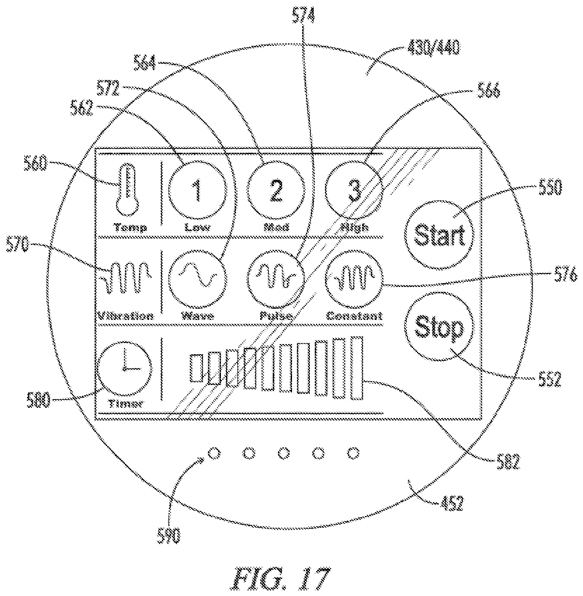

As shown in FIG. 17, the upper end of the control unit 140 comprises the LCD panel 430 and the overlying touch panel 440. The LCD panel displays a plurality of icons to convey information to a user regarding the operational mode of the vibration and heat generation apparatus 100 and to indicate to a user where to touch the touch panel to control the operation of the vibration and heat generation apparatus.

In the illustrated embodiment, a right hand portion of the LCD panel 430 displays a "Start" icon 550 and a "Stop" icon 552. Each icon represents a respective touch active portion of the overlying touch panel 440 such that touchingthe area of the "Start" icon activates the vibration and heat generation apparatus and touching the area of the "Stop" icon deactivates the vibration and heat generation apparatus. Although the vibration and heat generation apparatus is deactivated, the power remains on to provide an active display until the first pushbutton switch is pushed to turn off the power. When the Start icon is touchedto activate the apparatus, the display brightens (temporarily) to indicate that the apparatus is active.

The LCD panel 430 further displays a temperature icon 560 (represented by a thermometer symbol and the underlying letters "Temp." Three temperature selection icons are aligned with the temperature icon. Each temperature selectionicon corresponds to a touch active area of the overlying touch panel 440. A first temperature selection icon 562 is labeled with "1" and is further identified with "Low." A second temperature selection icon 564 is labeled with "2" and is further identified with "Med." A third temperature selection icon 564 is labeled with "3" and is further identified with High."

When the control unit 140 is first turned on and the start icon 550 is touched, no heating mode is selected. Touching the area of the first temperature selection icon 562 activates the "Low" heat mode icon and selects a temperature setting of approximately 42 degrees Celsius (approximately 108 degrees Fahrenheit). A ring around the first temperature selection icon is illuminated on the underlying LCD panel 430 to indicate that the low temperature range is selected. Touching the area of the first temperature selection icon when the ring is illuminated turns off the low heat mode. Touching the area of the second temperature selection icon 564 activates the "Med" heat mode icon and selects a temperature setting of approximately 50 degrees Celsius (approximately 122 degrees Fahrenheit). A ring around the second temperature selection icon is illuminated on the underlying LCD panel 430to indicate that the medium temperature range is selected. Touching the area of the second temperature selection icon when the ring is illuminated turns off the medium heat mode.

Touching the area of the third temperature selection icon 566 activates the "High" heat mode icon and selects a temperature setting of approximately 60 degrees Celsius (approximately 140 degrees Fahrenheit). A ring around the thirdtemperature selection icon is illuminated on the underlying LCD panel 430 to indicate that the high temperature range is selected. Touching the area of the third temperature selection icon when the ring is illuminated turns off the high heat mode.

Touching the stop icon area of the touch panel 440 clears any selected temperature selection. In operation, the control unit 140 monitors the resistance of the thermistor 360 and turns the heat generation unit 130 off and on based on the resistance. For example, when the "Low" heat setting is selected, the control unit detects when the thermistor becomes sufficiently hot (e.g., approximately 42 degrees Celsius) such that the resistance of the thermistor decreases below approximately 48,900 ohms. The control unit turns the heat generation unit off. The control unit continues to monitor the resistance of the thermistor while the thermistor cools and the resistance of the thermistor increases. When the thermistor is sufficiently cool (e.g., at a temperature below approximately 37 degrees Celsius) and the resistance of the thermistor increases above approximately 59,900 ohms, the heat generation unit is turned back on. The control unit operates in a similar manner for the other two temperature settings. For example, when the "Med" heat setting is selected, the control unit turns off the heat generation unit when the resistance of the thermistor decreases below approximately 35,900 ohms (corresponding to a temperature of approximately 50 degrees Celsius) and turns the heat generation unit back on when the resistance of the thermistor increases above approximately 48,900 ohms (corresponding to a temperature of approximately 42 degrees Celsius. When the "High" heat setting is selected, the control unit turns off the heat generation unit when the resistance of the thermistor decreases below approximately 24,750 ohms (corresponding to a temperature of approximately 60 degrees Celsius) and turns the heat generation unit back on when the resistance of the thermistor increases to above approximately 32,000 ohms (corresponding to a temperature below approximately 53 degrees Celsius).

The LCD panel 430 further displays a vibration selection icon 570 (represented by a waveform symbol and the underlying word "Vibration." Three vibration selection icons are aligned with the vibration icon. Each vibration selection icon corresponds to a touch active area of the overlying touch panel 440. A first vibration selection icon 572 is labeled with a first waveform icon and is further identified with "Wave." A second vibration selection icon 574 is labeled with a second waveform icon and is further identified with "Pulse." A third vibration selection icon 576 is labeled with a third waveform icon and is further identified with "Constant."

In the illustrated embodiment, when the control unit 140 is first turned on and the start icon 550 is touched, no vibration mode is selected. Touching the area of the first vibration selection icon 572 activates the wave vibration mode in which the four vibration pods 120, 122, 124, 126 are turned on in a selected sequence. A ring around the first vibration selection icon is illuminated on the underlying LCD panel 430 to indicate that the wave vibration mode is selected. Inone embodiment, the selected sequence of the wave vibration mode comprises turning on the first vibration pod for approximately one-quarter second; then turning off the first vibration pod and turning on the second vibration pod for approximately one-quarter second; then turning off the second vibration pod and turning on the third vibration pod for approximately one-quarter second; then turning off the third vibration pod and turning on the fourth vibration pod for approximately one-quarter second. The next sequence is started by turning off the fourth vibration pod and turning on the first vibration pod for approximately one-quarter second and repeating the foregoing steps. Rather than repeating the same sequence, subsequent sequences may turn the vibration pods on and off in a different order. Multiple vibration pods may also be turned on at the same time. The sequence orsequences are repeated as long as the control unit remains in the wave vibration mode. Touching the area of the first vibration selection icon when the ring is illuminated turns off the wave vibration mode.

Touching the area of the second vibration selection icon 574 activates the pulse vibration mode icon 574. A ring around the second vibration selection icon is illuminated on the underlying LCD panel 430 to indicate that the pulse vibrationmode is selected. In one embodiment, in the pulse vibration mode, the four vibration pods 120, 122, 124, 126 are turned on at the same time for a predetermined duration (e.g., approximately one-half second), and then turned offat the same time for a predetermined duration (e.g., approximately one-half second). The sequence of "all on" followed by "all off" is repeated as long as the control unit remains in the pulse vibration mode. Touching the area of the secondvibration selection icon when the ring is illuminated turns off the pulse vibration mode.

Touching the area of the third vibration selection icon 576 activates the constant vibration mode icon 574. A ring around the third vibration selection icon is illuminated on the underlying LCD panel 430 to indicate that the constant vibration mode is selected. In one embodiment, the four vibration pods 120, 122,124, 126 are operated continuously as long as the constant vibration mode is selected. Touching the area of the third vibration selection icon when the ring is illuminated turns off the constant vibration mode. Touching the stop icon 552 turns off the currently selected temperature mode and the currently selected vibration mode.

Any of the three vibration modes can be selected in combination with any of the three heat modes. Furthermore, a vibration mode may be selected withoutselecting a heat mode; and a heat mode may be selected without selecting a vibration mode.

The display panel 430 further displays a timer icon 580 represented by a solid circle and the underlying word "Timer." The timer icon is aligned with a sequence of 10 vertical timer bar icons 582 with increasing heights. Each timer bar icon represents an amount of time for which the vibration and heating apparatus 100 operates at the current vibration mode and heat mode settings before turning off automatically. For example, each timer bar icon may represent 2 minutes of remaining time such that when all bars are active, approximately 20 minutes of time remains before the apparatus turns off automatically. The tallest (right-most) timer bar is turned off at the end of approximately 2 minutes to indicate that only approximately 18 minutes remain. Each timer bar is sequentially turned off in similar intervals until the shortest (left-most) timer bar is turned off and the overall operation of the vibration and heat generation apparatus is stopped. The area of the timer bars is touch active such that any portion of the area of the timerbars can be touched at any time to reset the timer to the full twenty minutes. Thetimer bars are deactivated by touching the "Stop" icon 552. Touching the "Start" icon 550 restarts the timer at 20 minutes (all timer bars illuminated).

Although not part of either the LCD panel 430 or the touch panel 440, a plurality of display ports 590 (e.g., five display ports) are formed in the bezel 450. The display ports are aligned with a corresponding plurality of light emitting diodes (LEDs) 592 on the second PCB 420. The five LEDs are selectively illuminated to indicate the current charge on the LiPo battery 408. For example, all five LEDs are illuminated to indicate a fully charged battery. One LED at a time is turned off as the charge of the battery decreases. The last illuminated LED may be illuminated in a different color (e.g., red versus green) to indicate that the batteryneeds to be recharged.

The control unit 140 further includes the first conventional pushbutton switch 422 located on the perimeter of the control unit just below the LCD display 430 and touch panel 440 and facing the front of the vibration and heat generation apparatus 100. The first pushbutton switch operates as a master on/off switch to enable a user to operate the switch to turn the vibration and heat generation apparatus off to conserve the energy stored in the battery. The user operates thefirst pushbutton switch to turn the vibration and heat generation apparatus on such that the LCD display and the touch panel are activated to respond to touch commands as described above. The control unit further includes the second conventional pushbutton switch 424 located on the perimeter of the control unit just below the LCD panel and the touch panel and facing the right of the vibrationand heat generation apparatus. The second pushbutton switch provides a signal to the control unit to selectively dim the LCD panel to reduce energy consumptionwhen full brightness is not required. The activation of the second pushbutton switch also disables the touch panel from being responsive to touching by a user. Thus, any inadvertent touching of the touch panel will not change the mode of operation of the vibration and heat generation apparatus. In the illustrated embodiment, the LCD panel is automatically dimmed and the touch panel is automatically disabled after a short period of no touching by the user. For example, the LCD panel is dimmed and the touch panel is disabled after approximately 5 seconds of no touching by the user.

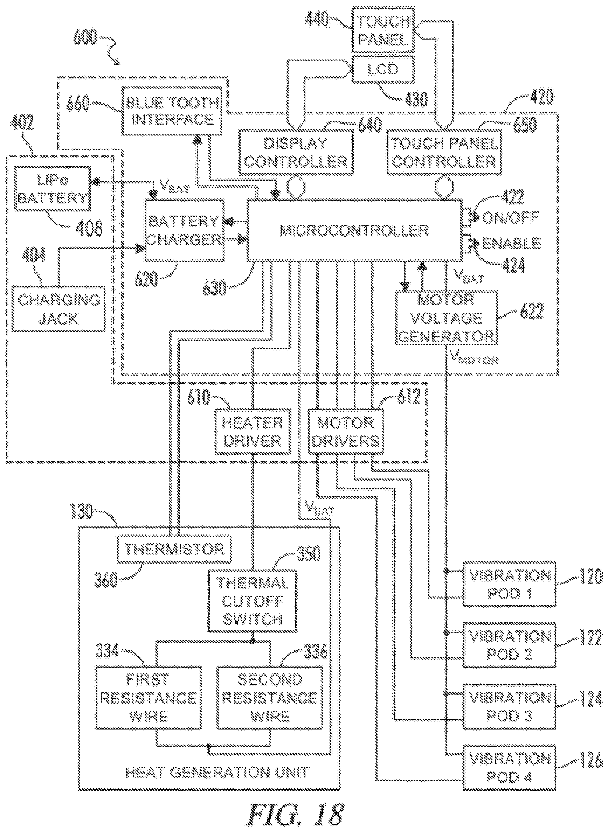

FIG. 18 illustrates a block diagram 600 of the electrical circuitry of the vibration and heat generation apparatus 100. In FIG. 18, previously identified components are numbered as before. The first PCB 402 and the second PCB 420 are illustrated in dashed lines to encompass the components on each PCB. The locations of the various components can vary in other embodiments. For example, the LiPo battery 408 and the charging jack 404 are shown as being part of the first PCB as described above. In the illustrated embodiment, the first PCB includes a heater driver 610 and motor drivers 612. In the illustrated embodiment, the heater driver and each of the four motor drivers comprises a power MOSFET that provides a current return path to ground when the respective driver is activated. In the illustrated embodiment, the battery LiPo battery is charged by a battery charger circuit 620, which is located on the second PCB. The battery charger circuit receives power from a conventional wall adapter (not shown) and charges the LiPo battery to approximately 16.8 volts. A second power control circuit ("motor voltage generator") 622 converts the battery voltage to approximately volts to drive the vibration motors 120, 122, 124, 126. In the illustrated embodiment, the motor voltage generator is also located on the second PCB. Although not shown in FIG. 18, the second PCB also includes circuitry to convert the battery voltage a supply voltage for the digital electronics circuitry. For example, a conventional 5-volt three-terminal voltage regulator (e.g., a Holtek HT7550-1) is suitable.

The second PCB 420 includes a microcontroller 630 that controls the operation of the other components on the second PCB and the first PCB 402. Forexample, the microcontroller in the illustrated embodiment is a commercially available 44-pin microcontroller that runs a conventional 8051 instruction set. One such microcontroller is an SN8F5707 microcontroller from Sonix in Taiwan. The microcontroller generates control signals to and receives feedback signals from the battery charger circuit 620 to control the charging of the LiPo battery 408. Themicrocontroller also controls the operation of the motor voltage generator 622 in asimilar manner. The microcontroller controls the heater driver 610 and the motordrivers 612 in response to commands received from a user. The microcontroller monitors a voltage responsive to the resistance of the thermistor 360 and selectively turns on and turns off the heater driver to maintain the temperature of the heat generation unit 130 within a selected temperature range.

The microcontroller 630 also controls the information displayed on the LCD panel 430 via a display controller 640. The microcontroller sends signals to the display controller representing the information to be displayed. The display controller receives the signals and generates the required command and data signals to the LCD to properly display the information. As discussed above, the displayed information includes the start and stop icons, the temperature icon with the three level icons, the vibration icon with the three vibration mode icons, and the timer icon with the 10 time bars. The control of an LCD is well-known in the art and is not described in detail herein. In the illustrated embodiment, the display controller is incorporated into the microcontroller. In other embodiments, the display controller may be a separate controller.

The microcontroller 630 receives signals from the touch panel 440 via a touch panel controller 650, which is located on the second PCB 420 in the illustrated embodiment. In the illustrated embodiment, the microcontroller communicates with the touch panel controller via a conventional 1²C bus. The microcontroller is responsive to signals from the touch panel controller that represent touching of the touch panel in areas corresponding to the icons displayed on the underlying LCD panel 430. The microcontroller is not responsive to touching of areas of the touch panel that do not correspond to a displayed icon. In the illustrated embodiment, the touch panel controller comprises a YS812A touch sensing microcontroller, which is commercially available from Taiwan Hui Electronics Co., Ltd., in Taiwan.

As discussed above, the microcontroller 630 is also responsive to the first pushbutton switch 422 and the second pushbutton switch 424. When the microcontroller is off and the first pushbutton switch is activated, the microcontroller awakens from a low power mode and generates the signals required to display the icons on the LCD panel 430. The microcontroller waits forsignals from the touch panel 440 via the touch panel controller 650. If a touch signal is received corresponding to the location of the start icon, the microcontroller becomes responsive to the touch signals from the heat selection icons and the vibration selection icons as described above. When the first pushbutton switch is activated while the microcontroller is active, the microcontroller turns off all functions and reenters the low-power state.

The microcontroller 630 is also responsive to the second pushbutton switch 424. Each time the second pushbutton switch is activated, the main controller toggles between a first display state and a second display state. In the first display state, the microcontroller sends a command to reduce the brightness of the LCD panel 430. In the first display state, the microcontroller is not responsive to any touch signals from the touch panel 440 via the touch panel controller 650. When the second pushbutton switch is activated when the microcontroller is in the first display state, the microcontroller responds by switching to the second display state wherein the microcontroller sends a command to increase the brightness of the icons of the LCD panel. While in the second display state, the microcontroller is responsive to touch signals from the touch panel via the touch panel controller. Inthe illustrated embodiment, the microcontroller automatically reenters the first display state after a selected period of inactivity (e.g., approximately 5 seconds) when the user does not touch an active portion of the touch panel. In the first display state, the reduction in brightness of the LCD saves energy; and the microcontroller is not responsive to any inadvertent touching of the touch panel.

The microcontroller 630 further sends commands to the LCD panel 430 to cause the LCD panel to display selected graphics as described above. In addition to sending commands to generate the static display icons shown in FIG. 17, the microcontroller also sends commands to selectively illuminate the ring icons that represent the current selected operational state (e.g., temperature setting low, medium or high; and vibration setting wave, pulse or constant). The microcontroller also updates the timer bar icons to display the remaining time before the microcontroller automatically turns off.

The microcontroller 630 receives commands from the touch panel 440 via the touch panel controller 650 when a user touches an active area of the touch panel. The microcontroller is responsive to the received commands to selectively control the operations of the four vibration pods 120, 122, 124, 126 and to controlthe operation of the heat generation unit 130.

The microcontroller 630 controls the first vibration pod 120 by selectively providing the motor voltage (e.g., approximately 12 volts DC) to the first vibration pod. In the illustrated embodiment, the microcontroller activates one or more of the motor drivers 612 to provide respective return paths to ground. The other three vibration pods 122, 124, 126 are controlled in a similar manner. The microcontroller controls the heat generation unit 130 by selectively providing the battery voltage (e.g., approximately 16.8 volts DC) to the heat generation unit. In the illustrated embodiment, the microcontroller activates the heater driver 610 to provide a return path to ground. The microcontroller is responsive to the resistance of the thermistor 360 to maintain the temperature within a range selected by the currently active temperature mode. As noted above, the thermal cutoff switch 350embedded in the heat generation unit independently opens the current path to theheat generation unit if the temperature of the heat generation unit exceeds approximately 80 degrees Celsius.

As further shown in FIG. 18, the vibration and heat generation apparatus 100 may also be controlled by a Bluetooth interface 660 coupled to a smartphone (not shown) or other device having a Bluetooth compatible interface. For example, in one embodiment, the Bluetooth interface is connected to the microcontroller 630 to send commands to and to receive information from the microcontroller. The Bluetooth interface is controlled by an application (App) running on the smartphone (or other device) that presents a user with a display screen having icons corresponding to the icons shown in FIG. 17. When a user touches the icons on the smartphone display, the commands are sent to the microcontroller via the coupled Bluetooth interfaces to control the microcontroller in a manner corresponding to the control of the microcontroller by the touch panel controller 650. The microcontroller responds by selecting the requested mode and by sending a confirmation to the smartphone App that the command has been received and has been implemented on the vibration and heat generation apparatus. The Bluetooth interface is particularly useful when the vibration and heat generation unit is positioned on a user's body in a location where the LCD display 430 is not easily viewed by the user.

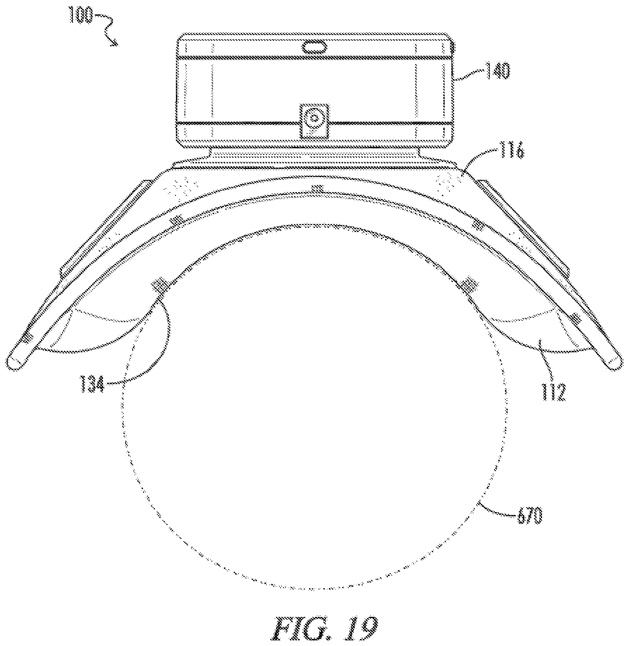

As shown in FIG. 19, the vibration and heat generation apparatus 100 is sufficiently flexible to bend around a generally cylindrical object 670 such as, for example, a human limb or joint (represented in dashed lines). The flexible lower bag-like structure 112 readily conforms to the contours of the limb or joint. The upper support structure 116 forms the outer boundary of the bent apparatus and positions the vibration pods and heat generating unit (within the enclosure 110) against the joint or limb receiving therapy. In addition to having an overall size and shape resembling a conventional flattened ice bag, the vibration and heat generation apparatus conforms to a human body part in a manner similar to an ice bag.

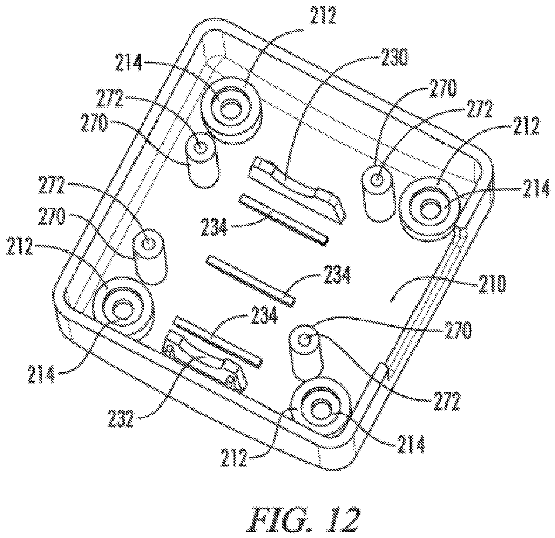

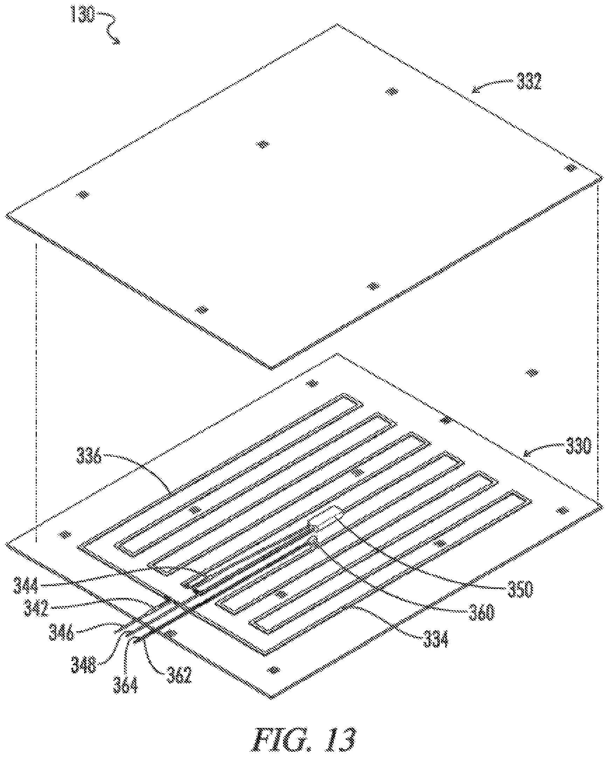

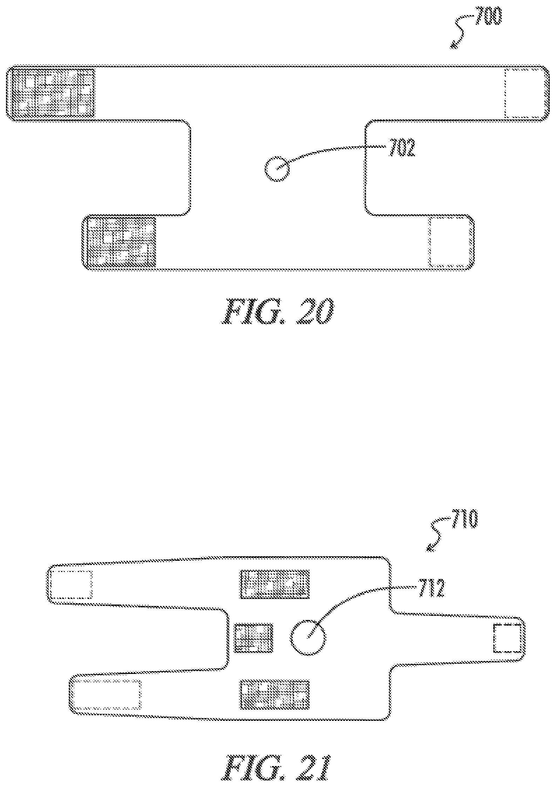

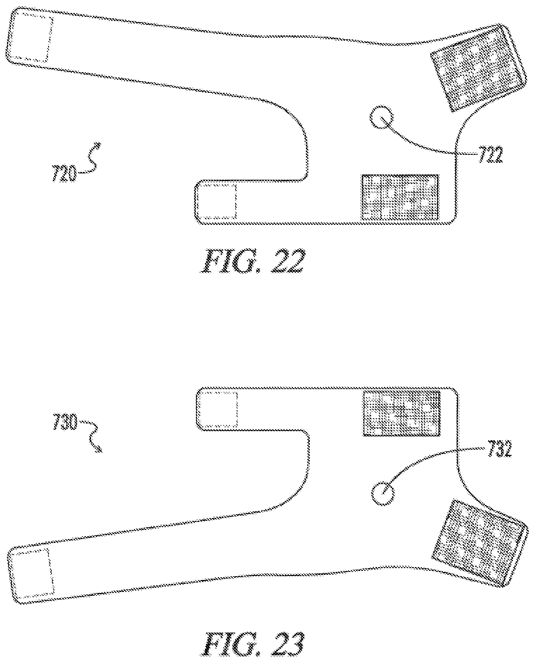

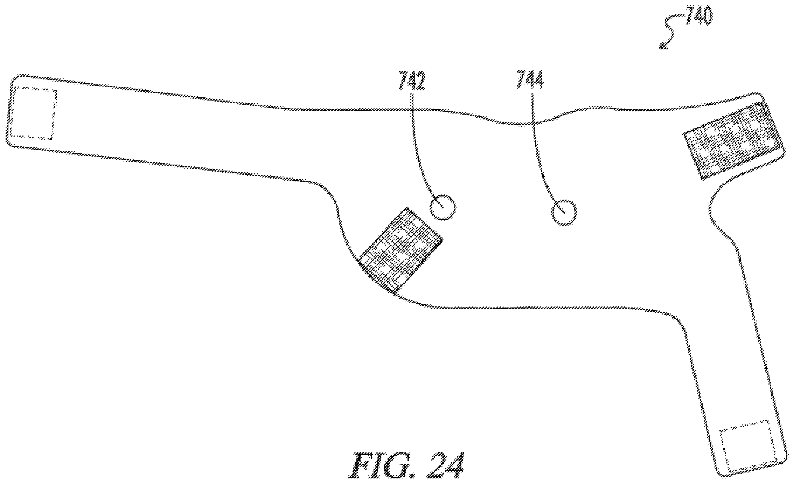

The vibration and heat generation apparatus 100 disclosed herein is configured for use with compression wraps that are used to apply compression toan ice bag positioned against a portion of a mammalian (e.g., human) body to provide therapeutic cooling. Such compression wraps are disclosed in US Patent No. 9,289,323, for "Ice Bag with Air Release Valve for Therapeutic Treatment, which issued on March 22, 2016. Figures 12-15 of the referenced patent illustrate compression wraps used to apply compression to an ice bag applied to a person's hip (Figure 12), to a person's knee (Figure 13), to a person's left shoulder (Figure 14) and to a person's right shoulder (Figure 15). Figure 16 of the referenced patent illustrates a compression wrap used to apply compression to a first ice bag applied to the front of a person's left shoulder and to apply compression to a second ice bag applied to the back of a person's left shoulder. Figures 17A and 17B of the referenced patent illustrate the application of two ice bags to a person's left shoulder using the compression wrap of Figure 16. The hip compression wrap of Figure 12 of the referenced patent is reproduced herein as a hip compression wrap 700 of FIG. 20. The hip compression wrap includes a circular bore 702 sized to receive the neck of the icebag described in the referenced patent. The knee compression wrap of Figure 13 of the referenced patent is reproduced herein as a knee compression wrap 710 of FIG. 21 having a circular bore 712 sized to receive the neck of the ice bag described in the referenced patent. The left shoulder compression wrap of Figure 14 of the referenced patent is reproduced herein as a left shoulder compression wrap 720 of FIG. 22 having a circular bore 722 sized to receive the neck of the ice bag described in the referenced patent. The right shoulder compression wrap of Figure 15 of the referenced patent is reproduced herein as a right shoulder compression wrap 730 of FIG. 23 having a circular bore 732 sized to receive the neck of the ice bag described in the referenced patent. The two icebag version of the left shoulder compression wrap of FIG. 16 of the referenced patent is reproduced herein as a compression wrap 740 of FIG. 24 having a first circular bore 742 sized to receive the neck of a first ice bag described in the referenced patent and having a second circular bore 744 sized to receive the neck of a second ice bag described in the referenced patent.

The cylindrical control unit 140 of the vibration and heat generation apparatus 100 has a shape and size selected to resemble an ice bag, such as, forexample, the ice bag illustrated in the above-referenced US Patent No. 9,289,323. The selected shape and size enables the vibration and heat generation unit to beoperable in combination with each of the compression wraps. The cylindrical control unit has a diameter of between about 50 millimeters and approximately 100 millimeters. For example in the illustrated embodiment, the control unit has a diameter of approximately 94 millimeters. The cylindrical bores in the existing compression wraps have diameters of approximately 45 millimeters. The material around the cylindrical bores easily stretches to accommodate the control unit andto hold the control unit snugly thereafter. The sizes of the cylindrical control unit and the sizes of the cylindrical bores can be varied; however, the illustrated dimensions provide a combination of sizes wherein the upper surface of the control unit has a sufficiently large size to accommodate the display and touch panel withicons of sufficient size to be easily manipulated while being sufficiently small to be inserted into a cylindrical bore that is able to receive and restrain the neck of a conventional ice bag or the ice bag shown in the referenced US Patent No. 9,289,323. By selecting the diameter of the control unit to be in a range of approximately 1.5 times to 3 times the diameter of the circular bore in a compression wrap, the compression wrap is able to stretch by a sufficient amountto accommodate the control unit without damaging the compression wrap and to exert a sufficient force on the control unit to secure the vibration and heat generation unit to the compression wrap while the compression wrap is being secured to the selected limb or joint of a person as described below.

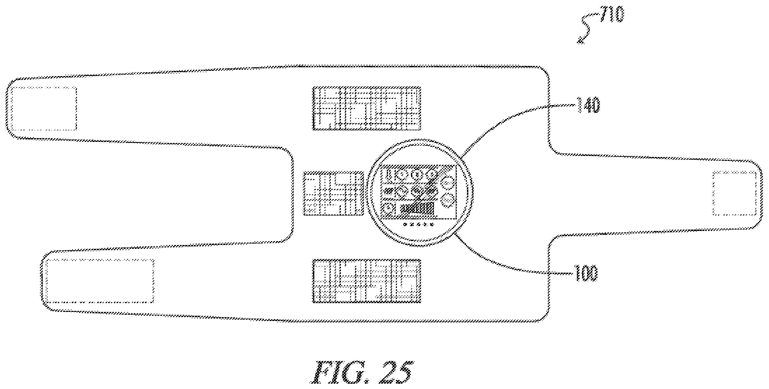

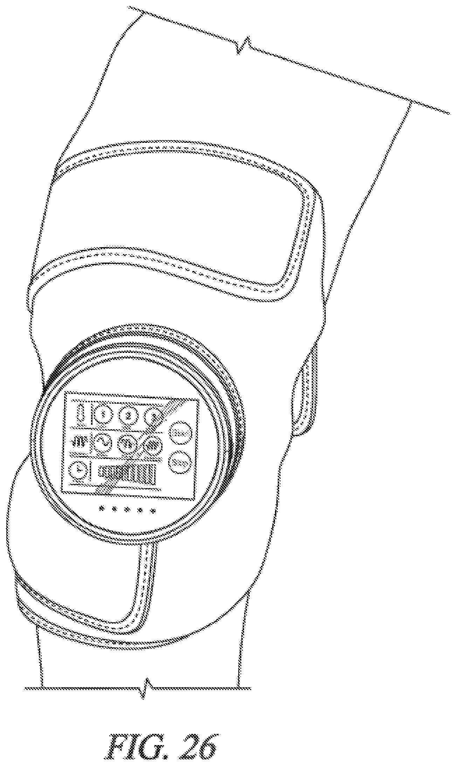

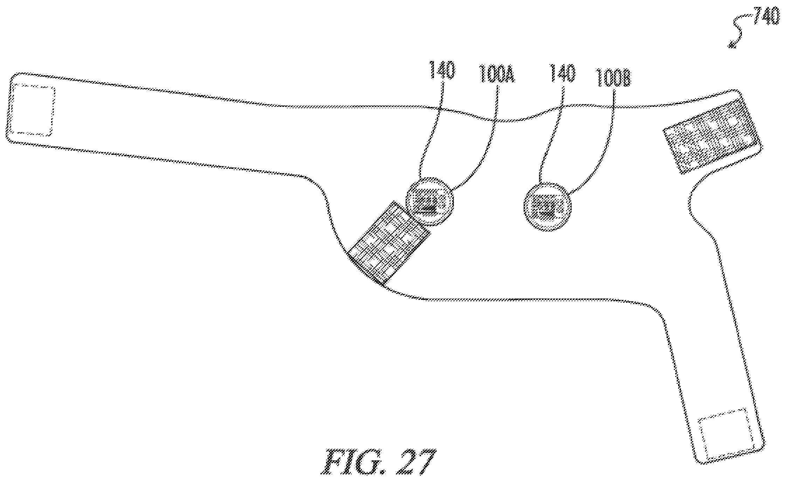

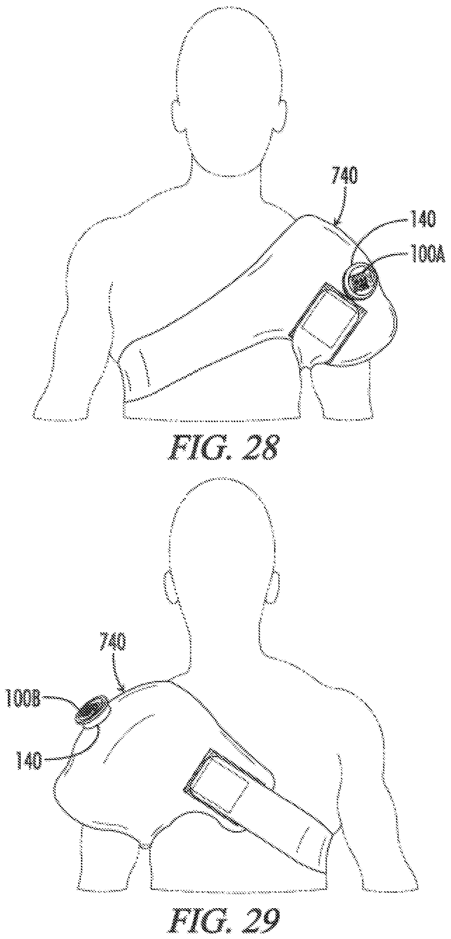

The control unit 140 of the vibration and heat generation apparatus 100 is inserted through the respective circular bore of one of the compression wraps of FIGS. 19-23. For example, FIG. 25 illustrates the vibration and heating apparatusin combination with the knee compression wrap 710 of FIG. 21 to apply vibration and heat to a person's knee. FIG. 26 illustrates the compression wrap and the vibration and heat generation apparatus applied to a knee. FIG. 27 illustrates a first vibration and heat generation apparatus 100A and a second vibration and heat generation apparatus 100B in combination with the compression wrap 740 of FIG. 23 to apply vibration and heat to the front and rear portions of a person's left shoulder. FIG. 28 illustrates a front view showing the compression wrap and the first vibration and heat generation apparatus on the person's left shoulder. FIG. 29 illustrates a rear view of the compression wrap and the second vibration and heat generation apparatus on the person's left shoulder.

The vibration and heat generation apparatus 100 described herein advantageously allows a person having a compression wrap useable with an ice bag for therapeutic cooling to remove the ice bag and install control unit 140 of the vibration and heat generation apparatus into the opening that receives the neck of the ice bag to provide therapeutic vibration and heat using the same compression wrap. Accordingly, a person does not have to have a separate compression wrap for each type of therapeutic treatment.

As various changes could be made in the above constructions without departing from the scope of the invention, it is intended that all the matter contained in the above description or shown in the accompanying drawings shall be interpreted as illustrative and not in a limiting sense. The inventions is defined by the appended claims

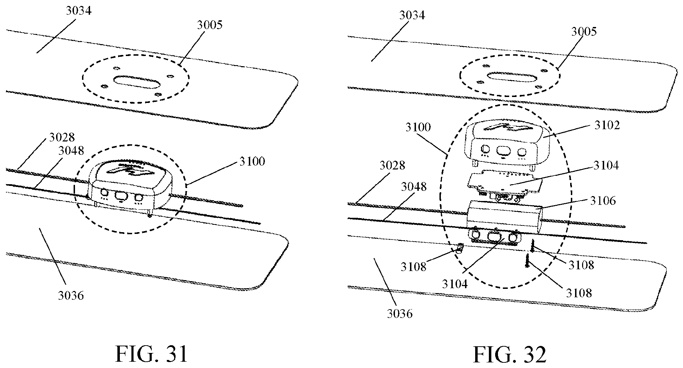

In another embodiment, the vibration and heat generation apparatus 100 disclosed herein can be configured for use with a temperature therapy device including a bladder that can be used to apply compression against a portion of a mammalian (e.g., human) body to provide thermal and vibration therapy. An example of such a temperature therapy device is disclosed in US Patent App. 17/384,501, for "System for Mounting Inelastic Components to a Flexible Material to Apply Compressive and Thermal Therapy", which was filed on July 23, 2021. Figures 12-13F of the referenced patent application illustrate the example temperature therapy device including a bladder used to apply compression to a person's body part (e.g., knee, although in other embodiments similar therapy). Such an example configuration is shown in FIGS. 31-32 and discussed in detail below.

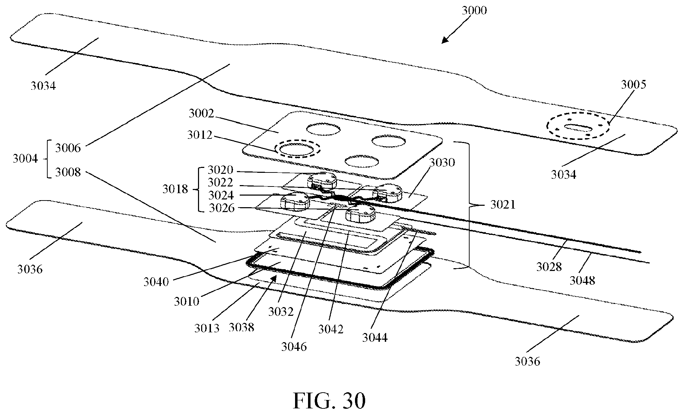

Referring to Figure 30, there is shown an exploded view of a vibration, thermal and compression device, according to some embodiments. The vibration, thermal and compression device 3000 can be similar to the vibration and heat generation apparatus 100 described above, e.g., described in FIGS. 1-18, but can also include a bladder 3002 and a multi-layer retention mechanism 3004. As shown, in some embodiments, the multi-layer retention mechanism can include a top layer 3006 and a bottom layer 3008. In some embodiments, the top layer 3006 can include an elastic material. In some embodiments, the bottom layer 3008 can include an inelastic material. In some embodiments, the bladder 3002 can be positioned between the top layer 3006 and the bottom layer 3008. In some embodiments, the bladder 3002 can enable the vibration, thermal and compression device 3000 to uniformly wrap around a body part of the user, e.g., via the bottom layer 3008, and allow the vibration, thermal and compression device 3000 to uniformly contact the users body part, e.g., via a silicone overmold insert 3010 coupled to the bottom layer 3008. In an example, portions of the bladder 3002 can be inflated and, once inflated, the bladder 3002 can compress against the bottom layer 3008. Upon compression, the pressure applied by the bladder 3002 to the bottom layer 3008 can allow for the bottom layer 3008 to uniformly surround the user's body part. The bladder 3002, can also apply pressure to a silicone overmold insert 3010 which can be part of and/or coupled to the bottom layer 3008. In an example, the bladder 3002 can apply pressure to the silicone overmold insert 3010 such that the silicone overmold insert 3010 uniformly contacts the skin of the user's body part. In some embodiments, the bladder 3002 can include one or more openings 3012 that correspond to the one or more vibration pods 3020, 3022, 3024, 3026 of a plurality of vibration elements 3018. In some embodiments, the bladder 3002 can be bonded to an outer perimeter 3013 of the bottom layer 3008, in some cases solely to the outer perimeter 3013 (or a portion thereof), such that the bladder 3002 is not bonded to the bottom layer 3008 at surfaces within the perimeter of the bottom layer 3008. In general, any suitable attachment technique can be used to secure the bladder 3002 and the bottom layer 3008. In an example, the bladder 3002 can be bonded to the bottom layer 3008 via an adhesive. In some examples, the bladder 3002 can be sewn directly to the outer perimeter of the bottom layer 3008. In an example, bonding the bladder 3002 to the outer perimeter of the bottom layer 3008 can enable the bladder 3002 to wrap around the user's body part (e.g., a user's knee) when the bladder 3002 inflates, as opposed to lifting off the user's body part and only constricting around the user's body part. In some embodiments, the bladder 3002 can be configured to allow the silicone overmold insert 3010 to uniformly contact the skin of the user's body part eliminating any air gaps or reducing the number of air gaps between the silicone overmold insert 3010 and the skin of the user. In some embodiments, the bladder 3010 and the bottom layer 3008 can include a zipper attachment that is configured to attach and/or secure the bladder 3010 to the bottom layer 3008. In some examples, the zipper attachment can allow for the bladder 3010 to be removed, e.g., after unzipping the zipper attachment between the bladder 3010 and bottom layer 3008. In some embodiments, the silicone overmold insert 3010 can include and/or also be referred to herein as a molded silicone. In some embodiments, the bladder 3002 can also be referred to herein as a compressive element. In some examples, the bladder 3002 can include an inflatable bladder.

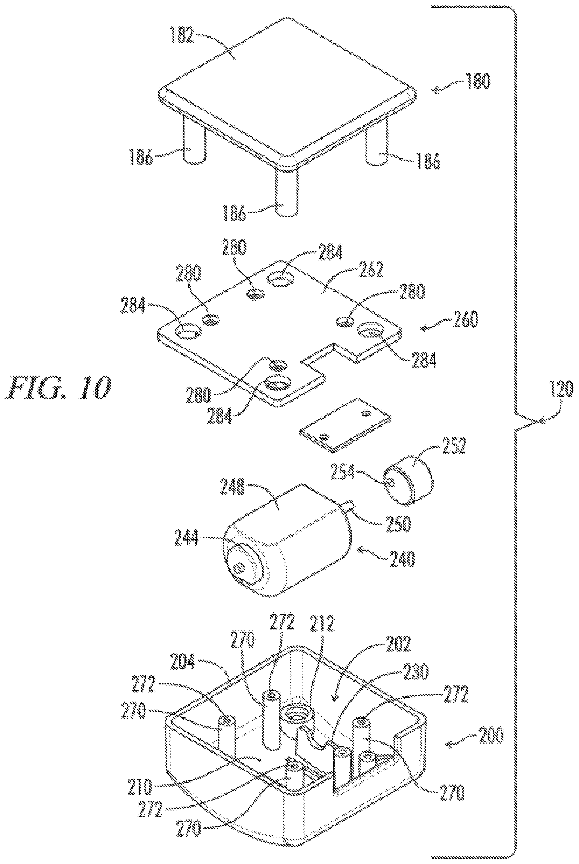

Referring again to FIG. 30, the vibration, thermal and compression device 3000 can also include a plurality of vibration elements. The plurality of vibration elements 3018 can be the same or similar to the plurality of vibration elements described in FIGS. 1-18 above. In an example, the plurality of vibration elements 3018 can include the vibration pods 3020-3026. In some examples, the vibration pods 3020-3026 are similar and/or the same to the vibration pods 120-126 described in FIG. 1-18 above (e.g., shown in FIG. 7 and described above). In some embodiments, although the vibration, thermal and compression device 3000 shown in FIG. 30 includes four vibration pods 3020-3026, the vibration, thermal and compression device 3000 is not limited to four vibration pods 3020-3026 and can include one or more vibration pods (e.g., one, two, four, six, eight, ten, or twenty vibration pods). In a further example, the vibration pods 3020-3026 can include the same or similar structures to those described in FIGS. 9-12. In an example, each of the vibration pods 3020-3026 can include the covers 180, 200, motor 240 and motor clamp plate 260, among other features, described in FIG. 10. In some embodiments, the plurality of vibration elements 3018 can include one or more wires 3028 for the vibration elements. In some examples, the one or more wires 3028 can be coupled to the vibration pods 3020-3026. In some examples, the one or more wires 3028 can couple the vibration pods to 3020-3026 a control module 3100 (e.g., described in FIG. 31, 32 below). In some examples, the one or more wires 3028 can allow for electronic coupling and/or communication between the vibration pods 3020-3026 and the control module 3100 of FIGS. 31 and 32. Each of the vibration pods 3020-3026 includes a bonding structure 3030, where the bonding structure 3030 is configured to bond and/or adhere the each of the vibration pods 3020-3026 to a thermal pad 3032 (e.g., the thermal pad described below). In some examples, the bonding structure 3030 can including an adhesive and/or tape.

Referring again to FIG. 30, the top layer 3006 and the bottom layer 3008 of the multi-layer retention mechanism 3004 can include various features. In some embodiments, the top layer 3004 and/or bottom layer 3006 can include a flexible fabric and/or an elastic material. In some embodiments, the top layer 3006 can include one or more top straps 3034. In some embodiments, the bottom layer 3008 can include one or more bottom straps 3036. In an example, the top layer 3004 and/or bottom layer 3006 can include polyester and/or spandex. In some embodiments, the top layer 3002 can include one or more cavities 3005 configured to receive the control module 3100 of FIGS. 31 and 32. In some embodiments, the bottom layer 3006 can include and/or be coupled to the silicone overmold insert 3010. In some embodiments, the silicone overmold insert 3010 can be configured to be placed on a user's body part (e.g., a knee region, a lower back region, an elbow region, etc.). In an example, the bottom layer 3008 can include a cavity 3038 configured for receiving the silicone overmold insert 3010. In some embodiments, the top layer 3006 can be bonded to the bottom layer 3008. In an example, the top layer 3006 can be bonded to the bottom layer 3008 via sewing, stitching, gluing, adhering, among other techniques and/or bonding processes. In an example, the top layer 3006 and to the bottom layer 3008 can be sewn, stitched, glued, and/or adhered together.

Referring again to FIG. 30, in some embodiments, the vibration, thermal and compression device 3000 can include a heat spreader 3040 disposed between the thermal pad and the silicone overmold insert. In some embodiments, the heat spreader 3040 can be configured to attach to the silicone overmold insert 3010. In some embodiments, the thermal pad 3032 can be configured to attach to the heat spreader 3040. In some embodiments, the thermal pad 3032 and/or heat spreader 3040, either together in combination or alone, can be configured to apply heat and/or cold therapy to a user's body part. In some embodiments, the thermal pad 3032 and/or heat spreader 3040, either together in combination or alone, can function to regulate the temperature of the hot or cold therapy based on received control instructions (e.g., from a mobile application-based controller, a computing device, a mobile computing platform, a client application execution thereon, etc.). In some embodiments, the thermal pad 3032 can include a curved structured, e.g., a letter "S" like structure or referred to herein as a S-structure 3042 (e.g., as shown in FIG. 30). In some embodiments, the S-structure 3042 of the thermal pad 3032 may not be included or used by the thermal pad 3032. In some examples, the thermal pad 3032 can include the heat generation unit 130 of FIGS. 13 and 14. In some embodiments, the thermal pad 3032 can include a first (lower) rectangular sheet of cloth 330 and a second (upper) rectangular sheet of cloth 332, as shown in the heat generation unit 130 of FIGS. 13 and 14. In some embodiments, the thermal pad 3032 can include input/output wires 3044 for controlling the thermal pad 3032. In some embodiments, the input/output wires 3044 can instead include copper covered with polyimide.

Referring again to FIG. 30, the vibration, thermal and compression device 3000 can also include a temperature sensor, as shown. In some embodiments, temperature sensor 3046 can be configured to detect the temperature of the vibration, thermal and compression device 3000 and provide feedback to the control module (e.g., the control module described in FIGS. 31 and 32). In some examples, the temperature sensor 3046 can be placed on and/or adjacent to the thermal pad 3032. In some examples, the temperature sensor 3046 can be configured to detect the temperature of the thermal pad 3032 and provide the control module with the detected temperature of the thermal pad 3032. In some examples, a temperature sensor wire 3048 can couple the temperature sensor 3046 to the control module 3100 of FIGS. 31 and 32 (e.g., allow for electronic coupling and/or communication between the temperature sensor 3046 and the control module 3100).

Referring again to FIG. 30, the vibration, thermal and compression device 3000 can also include a vibration, thermal and compressive element disposed between the top layer and bottom layer. In some embodiments, the vibration, thermal and compressive element 3021 can include the bladder 3002, the plurality of vibration elements 3018, the thermal pad 3032, the heat spreader 3040 and the silicone overmold insert 3010, among other components. In some embodiments, the vibration, thermal and compressive element 3021 can be configured such that, upon activation of the vibration, thermal and compressive element 3021 a vibration force can be applied (e.g., from the plurality of vibration elements 3018), a thermal therapy can be applied (e.g., from the thermal pad 3032, heat spreader 3040 and/or silicone overmold insert 3010 together or from each component), and a compressive force can be applied to the body surface of a user (e.g., via the bladder 3002). In some cases, the compressive element (e.g., the bladder 3002) can curve to more closely conform to the bottom layer 3008 to the user's body part.

Referring to FIGS. 31 and 32, an example control module for the vibration, thermal and compression device is shown, according to some embodiments. In general, the control module 3100 can be located in any suitable location and can control the device 3000 via either a hard wired connection or a wireless connection. In some embodiments, the control module 3100 can be placed and/or coupled to the vibration, thermal and compression device 3000 via the encircled location and/or the cavities 3005 shown in FIG. 30. In some embodiments, the control module 3100 can include an electronics housing 3102 and electronic parts 3104 inside the electronics housing 3100. In some embodiments, the electronic parts 3104 can include various electronics such as one or more microcontrollers, LEDs, sensors, push buttons, buttons, among other electronic parts. In some embodiments, the control module 3100 can be communicatively coupled to the vibration, thermal and compression device 3000 and also retained by the multi-layer retention mechanism 3002. In an example, the control module 3100 can be communicatively coupled to the vibration, via the wires 3028, 3046. In some examples, the wires 3028 can couple the vibration pods to 3020-3026 of FIG. 30 to the control module 3100. In some examples, a temperature sensor wire 3048 can couple the temperature sensor 3046 of FIG. 30 to the control module 3100. In some embodiments, the control module 3100 can include a power supply and/or power electronics 3106. In some examples, the power electronics 3106 can include a battery (e.g., a lithium ion battery). In some embodiments, the control module 3100 can include coupling features 3108. In some examples, the coupling features 3108 can include mechanical screws and/or mechanical features configured for coupling the electronics and/or other mechanical features of the control module 3100 to the electronics housing 3102. As used herein, the control module 3100 can also be referred to as an electronics box, collected electronics, electronics housing, among other terms. In some embodiments, the control module 3100 can include an air compressor configured to inflate and/or deflate the bladder 3002 of FIG. 30.

### Computer Systems

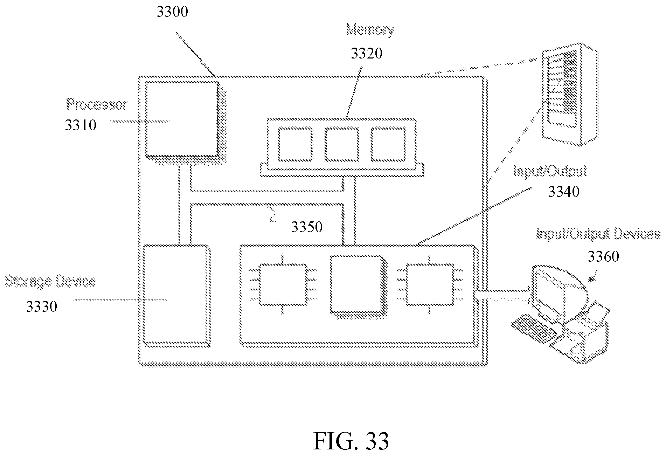

FIG. 33 is a block diagram of an example computer system 3300 that may be used in implementing the technology described in this document. General-purpose computers, network appliances, mobile devices, or other electronic systems may also include at least portions of the system 3300. The system 3300 includes a processor 3310, a memory 3320, a storage device 3330, and an input/output device 3340. Each of the components 3310, 3320, 3330, and 3340 may be interconnected, for example, using a system bus 3350. The processor 3310 is capable of processing instructions for execution within the system 3300. In some implementations, the processor 3310 is a single-threaded processor. In some implementations, the processor 3310 is a multi-threaded processor. The processor 3310 is capable of processing instructions stored in the memory 3320 or on the storage device 3330.

The memory 3320 stores information within the system 3300. In some implementations, the memory 3320 is a non-transitory computer-readable medium. In some implementations, the memory 3320 is a volatile memory unit. In some implementations, the memory 3320 is a non-volatile memory unit.

The storage device 3330 is capable of providing mass storage for the system 3300. In some implementations, the storage device 3330 is a non-transitory computer-readable medium. In various different implementations, the storage device 3330 may include, for example, a hard disk device, an optical disk device, a solid-date drive, a flash drive, or some other large capacity storage device. For example, the storage device may store long-term data (e.g., database data, file system data, etc.). The input/output device 3340 provides input/output operations for the system 3300. In some implementations, the input/output device 3340 may include one or more of a network interface devices, e.g., an Ethernet card, a serial communication device, e.g., an RS-232 port, and/or a wireless interface device, e.g., an 802.11 card, a 3G wireless modem, or a 4G wireless modem. In some implementations, the input/output device may include driver devices configured to receive input data and send output data to other input/output devices, e.g., keyboard, printer and display devices 3360. In some examples, mobile computing devices, mobile communication devices, and other devices may be used.

In some implementations, at least a portion of the approaches described above may be realized by instructions that upon execution cause one or more processing devices to carry out the processes and functions described above. Such instructions may include, for example, interpreted instructions such as script instructions, or executable code, or other instructions stored in a non-transitory computer readable medium. The storage device 3330 may be implemented in a distributed way over a network, for example as a server farm or a set of widely distributed servers, or may be implemented in a single computing device.

Although an example processing system has been described in FIG. 33, embodiments of the subject matter, functional operations and processes described in this specification can be implemented in other types of digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible nonvolatile program carrier for execution by, or to control the operation of, data processing apparatus. Alternatively or in addition, the program instructions can be encoded on an artificially generated propagated signal, e.g., a machine-generated electrical, optical, or electromagnetic signal that is generated to encode information for transmission to suitable receiver apparatus for execution by a data processing apparatus. The computer storage medium can be a machine-readable storage device, a machine-readable storage substrate, a random or serial access memory device, or a combination of one or more of them.

The term "system" may encompass all kinds of apparatus, devices, and machines for processing data, including by way of example a programmable processor, a computer, or multiple processors or computers. A processing system may include special purpose logic circuitry, e.g., an FPGA (field programmable gate array) or an ASIC (application specific integrated circuit). A processing system may include, in addition to hardware, code that creates an execution environment for the computer program in question, e.g., code that constitutes processor firmware, a protocol stack, a database management system, an operating system, or a combination of one or more of them.

A computer program (which may also be referred to or described as a program, software, a software application, a module, a software module, a script, or code) can be written in any form of programming language, including compiled or interpreted languages, or declarative or procedural languages, and it can be deployed in any form, including as a standalone program or as a module, component, subroutine, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable computers executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Computers suitable for the execution of a computer program can include, by way of example, general or special purpose microprocessors or both, or any other kind of central processing unit. Generally, a central processing unit will receive instructions and data from a read-only memory or a random access memory or both. A computer generally includes a central processing unit for performing or executing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device (e.g., a universal serial bus (USB) flash drive), to name just a few.

Computer readable media suitable for storing computer program instructions and data include all forms of nonvolatile memory, media and memory devices, including by way of example semiconductor memory devices, e.g., EPROM, EEPROM, and flash memory devices; magnetic disks, e.g., internal hard disks or removable disks; magneto optical disks; and CD-ROM and DVD-ROM disks. The processor and the memory can be supplemented by, or incorporated in, special purpose logic circuitry.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's user device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), e.g., the Internet.

The computing system can include clients and servers. A client and server are generally remote from each other and typically interact through a communication network. The relationship of client and server arises by virtue of computer programs running on the respective computers and having a client-server relationship to each other.

While this specification contains many specific implementation details, these should not be construed as limitations on the scope of what may be claimed, but rather as descriptions of features that may be specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In certain implementations, multitasking and parallel processing may be advantageous. Other steps or stages may be provided, or steps or stages may be eliminated, from the described processes. Accordingly, other implementations are within the scope of the following claims.

### Terminology

The phraseology and terminology used herein is for the purpose of description and should not be regarded as limiting.

Measurements, sizes, amounts, and the like may be presented herein in a range format. The description in range format is provided merely for convenience and brevity and should not be construed as an inflexible limitation on the scope of the invention. Accordingly, the description of a range should be considered to have specifically disclosed all the possible subranges as well as individual numerical values within that range. For example, description of a range such as 1-20 meters should be considered to have specifically disclosed subranges such as 1 meter, 2 meters, 1-2 meters, less than 2 meters, 10-11 meters, 10-12 meters, 10-13 meters, 10-14 meters, 11-12 meters, 11-13 meters, etc.

Furthermore, connections between components or systems within the figures are not intended to be limited to direct connections. Rather, data or signals between these components may be modified, re-formatted, or otherwise changed by intermediary components. Also, additional or fewer connections may be used. The terms "coupled," "connected," or "communicatively coupled" shall be understood to include direct connections, indirect connections through one or more intermediary devices, wireless connections, and so forth.

The term "approximately", the phrase "approximately equal to", and other similar phrases, as used in the specification and the claims (e.g., "X has a value of approximately Y" or "X is approximately equal to Y"), should be understood to mean that one value (X) is within a predetermined range of another value (Y). The predetermined range may be plus or minus 20%, 10%, 5%, 3%, 1%, 0.1%, or less than 0.1%, unless otherwise indicated.

The indefinite articles "a" and "an," as used in the specification and in the claims, unless clearly indicated to the contrary, should be understood to mean "at least one." The phrase "and/or," as used in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of or "exactly one of," or, when used in the claims, "consisting of," will refer to the inclusion of exactly one element of a number or list of elements. In general, the term "or" as used shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The use of "including," "comprising," "having," "containing," "involving," and variations thereof, is meant to encompass the items listed thereafter and additional items.

Use of ordinal terms such as "first," "second," "third," etc., in the claims to modify a claim element does not by itself connote any priority, precedence, or order of one claim element over another or the temporal order in which acts of a method are performed. Ordinal terms are used merely as labels to distinguish one claim element having a certain name from another element having a same name (but for use of the ordinal term), to distinguish the claim elements.

Having thus described several aspects of at least one embodiment of this invention, it is to be appreciated that various alterations, modifications, and improvements will readily occur to those skilled in the art. Such alterations, modifications, and improvements are intended to be part of this disclosure.

Accordingly, the foregoing description and drawings are by way of example only. The invention is defined in the appended claims.

## Claims

1. A therapeutic device (3000) .
for applying vibration, thermal and compressive therapy, the device comprising:
a top layer (3006);
a bottom layer (3008) adapted to contact a body surface of a user; and
a therapeutic element (3021)
disposed between the top layer and the bottom layer, the therapeutic element comprising
a vibration component (3018);
a thermal component (3032); and
a compression component,
wherein, upon activation of the therapeutic element: (i) the vibration component applies a vibration force, (ii) the thermal component applies a thermal therapy, and (iii) the compression component applies a compressive force,
**characterised in that**, the vibration component is bonded directly to the thermal component via a bonding structure (3030).

2. The device of claim 1, wherein the top layer comprises a flexible, elastic material.

3. The device of claim 1, wherein the bottom layer comprises an inelastic material; optionally wherein the inelastic material comprises molded silicone.

4. The device of claim 1, wherein the compression component comprises an inflatable bladder (3002); optionally wherein the device further comprises an air compressor adapted to selectively inflate the inflatable bladder; optionally wherein the air compressor is disposed within a control module 3100).

5. The device of claim 1, wherein the compression component is bonded to the bottom layer; optionally wherein the compression component is bonded to the bottom layer solely at the perimeter of the bottom layer.

6. The device of claim 1, wherein one or more of the vibration component, the thermal component, and the compression component are the same component.

7. The device of claim 1, wherein, upon activation of the therapeutic element, the compression component curves to more closely conform to the bottom layer.

8. The device of any one of claims 1 to 7, wherein the vibration component comprises a plurality of vibration elements (3020, 3022, 3024, 3026).

9. The device of claim 8, wherein the plurality of vibration elements comprises a plurality of vibration pods.

10. The device of claim 8, wherein the plurality of vibration elements are electrically coupled to a control module.

11. The device of claim 8, wherein the compression component comprises an inflatable bladder (3002).

12. The device of any one of claims 1 to 11, wherein the thermal component comprises at least one of a thermal pad (3032), a heat spreader (3040) or a silicone overmold insert (3010).

13. The device of claim 12, wherein the top layer comprises a flexible, elastic material.

14. The device of claim 12, wherein the vibration component comprises a plurality of vibration elements; optionally wherein the compression component comprises an inflatable bladder; optionally wherein upon activation of the therapeutic element: (i) the plurality of vibration elements apply a vibration force, (ii) at least one of the thermal pad, the heat spreader and the silicone overmold insert applies a thermal therapy, and (iii) the inflatable bladder applies a compressive force.

## Patentansprüche

1. Therapeutische Vorrichtung (3000) zur Anwendung von Vibrations-, thermischer und Kompressionstherapie, wobei die Vorrichtung umfasst:
eine obere Schicht (3006);
eine untere Schicht (3008), gestaltet zum Kontakt mit einer Körperoberfläche eines Anwenders; und
ein therapeutisches Element (3021), das zwischen der oberen Schicht und der unteren Schicht angeordnet ist, wobei das therapeutische Element umfasst
eine Vibrationskomponente (3018);
eine thermische Komponente (3032);
und
eine Kompressionskomponente,
wobei bei Aktivierung des therapeutischen Elements: (i) die Vibrationskomponente eine Vibrationskraft ausübt, (ii) die thermische Komponente eine thermische Therapie ausübt und (iii) die Kompressionskomponente eine Kompressionskraft ausübt, **dadurch gekennzeichnet, dass** die Vibrationskomponente über eine Verbindungsstruktur (3030) direkt an die thermische Komponente gebunden ist.

2. Vorrichtung nach Anspruch 1, wobei die obere Schicht ein flexibles elastisches Material umfasst.

3. Vorrichtung nach Anspruch 1, wobei die untere Schicht ein unelastisches Material umfasst; gegebenenfalls wobei das unelastische Material geformtes Silicon umfasst.

4. Vorrichtung nach Anspruch 1, wobei die Kompressionskomponente eine aufblähbare Blase (3002) umfasst;
wobei die Vorrichtung gegebenenfalls ferner einen Luftkompressor umfasst, der dafür gestaltet ist, die aufblähbare Blase selektiv aufzublähen; wobei der Luftkompressor gegebenenfalls innerhalb eines Steuermoduls (3100) angeordnet ist.

5. Vorrichtung nach Anspruch 1, wobei die Kompressionskomponente an die untere Schicht gebunden ist; wobei die Kompressionskomponente gegebenenfalls nur an dem Umfang der unteren Schicht an die untere Schicht gebunden ist.

6. Vorrichtung nach Anspruch 1, wobei eine oder mehrere von der Vibrationskomponente, der thermischen Komponente und der Kompressionskomponente die gleiche Komponente sind.

7. Vorrichtung nach Anspruch 1, wobei sich die Kompressionskomponente bei Aktivierung des therapeutischen Elements krümmt, um sich näher an die untere Schicht anzupassen.

8. Vorrichtung nach einem der Ansprüche 1 bis 7, wobei die Vibrationskomponente eine Vielzahl von Vibrationselementen (3020, 3022, 3024, 3026) umfasst.

9. Vorrichtung nach Anspruch 8, wobei die Vielzahl von Vibrationselementen eine Vielzahl von Vibrationskapseln umfasst.

10. Vorrichtung nach Anspruch 8, wobei die Vielzahl von Vibrationselementen elektrisch mit einem Steuermodul gekoppelt ist.

11. Vorrichtung nach Anspruch 8, wobei die Kompressionskomponente eine aufblähbare Blase (3002) umfasst.

12. Vorrichtung nach einem der Ansprüche 1 bis 11, wobei die thermische Komponente wenigstens eines von einem Thermokissen (3032), einem Wärmeverteiler (3040) und einem Silicon-umspritzten Einsatz (3010) umfasst.

13. Vorrichtung nach Anspruch 12, wobei die obere Schicht ein flexibles elastisches Material umfasst.

14. Vorrichtung nach Anspruch 12, wobei die Vibrationskomponente eine Vielzahl von Vibrationselementen umfasst; wobei die Kompressionskomponente gegebenenfalls eine aufblähbare Blase umfasst; wobei bei Aktivierung des therapeutischen Elements gegebenenfalls: (i) die Vielzahl von Vibrationselemente eine Vibrationskraft ausübt, (ii) wenigstens eines von dem Thermokissen, dem Wärmeverteiler und dem Silicon-umspritzten Einsatz eine thermische Therapie ausübt, und (iii) die aufblähbare Blase eine Druckkraft ausübt.

## Revendications

1. Dispositif thérapeutique (3000) destiné à appliquer une thérapie par vibration, thermique et compressive, le dispositif comprenant :
une couche supérieure (3006) ;
une couche inférieure (3008) adaptée pour entrer en contact avec une surface corporelle d'un utilisateur ; et
un élément thérapeutique (3021) disposé entre la couche supérieure et la couche inférieure, l'élément thérapeutique comprenant
un composant de vibration (3018) ;
un composant thermique (3032) ;
et
un composant de compression,
lors de l'activation de l'élément thérapeutique : (i) le composant de vibration appliquant une force de vibration, (ii) le composant thermique appliquant une thérapie thermique, et (iii) le composant de compression appliquant une force de compression, **caractérisé en ce que** le composant de vibration est lié directement au composant thermique par l'intermédiaire d'une structure de liaison (3030).

2. Dispositif selon la revendication 1, la couche supérieure comprenant un matériau élastique flexible.

3. Dispositif selon la revendication 1, la couche inférieure comprenant un matériau non élastique ; éventuellement, le matériau non élastique comprenant de la silicone moulée.

4. Dispositif selon la revendication 1, le composant de compression comprenant une vessie gonflable (3002) ; éventuellement, le dispositif comprenant en outre un compresseur d'air adapté pour gonfler sélectivement la vessie gonflable ; éventuellement, le compresseur d'air étant disposé à l'intérieur d'un module de commande (3100).

5. Dispositif selon la revendication 1, le composant de compression étant lié à la couche inférieure ; éventuellement, le composant de compression étant lié à la couche inférieure uniquement au niveau du périmètre de la couche inférieure.

6. Dispositif selon la revendication 1, un ou plusieurs du composant de vibration, du composant thermique et du composant de compression étant le même composant.

7. Dispositif selon la revendication 1, lors de l'activation de l'élément thérapeutique, le composant de compression s'incurvant pour épouser plus étroitement la forme de la couche inférieure.

8. Dispositif selon l'une quelconque des revendications 1 à 7, le composant de vibration comprenant une pluralité d'éléments de vibration (3020, 3022, 3024, 3026).

9. Dispositif selon la revendication 8, la pluralité d'éléments de vibration comprenant une pluralité de capsules de vibration.

10. Dispositif selon la revendication 8, les multiples éléments de vibration étant couplés électriquement à un module de commande.

11. Dispositif selon la revendication 8, le composant de compression comprenant une vessie gonflable (3002).

12. Dispositif selon l'une quelconque des revendications 1 à 11, le composant thermique comprenant au moins un parmi un patin thermique (3032), un dissipateur de chaleur (3040) ou un insert de surmoulage en silicone (3010).

13. Dispositif selon la revendication 12, la couche supérieure comprenant un matériau élastique flexible.

14. Dispositif selon la revendication 12, le composant de vibration comprenant une pluralité d'éléments de vibration ; éventuellement, le composant de compression comprenant une vessie gonflable ; éventuellement, lors de l'activation de l'élément thérapeutique : (i) la pluralité d'éléments de vibration applique une force de vibration, (ii) au moins un élément parmi le patin thermique, le dissipateur de chaleur et l'insert de surmoulage en silicone applique une thérapie thermique, et (iii) la vessie gonflable applique une force de compression.
